# EUROPEAN PATENT APPLICATION

(11) **EP 3 957 624 A1**
(43) Date of publication of application: **23.02.2022**
(21) Application number: 20191964.4
(22) Date of filing: 20.08.2020
(51) Int. Cl.: C07C 67/00, C07D 213/56, C07C 45/00, C07C 311/51, C07C 327/22, C07D 263/38, C07C 67/30, C07C 231/12, C07C 253/30

(54) **METHOD FOR THE PREPARATION OF ARYL OR HETEROARYL SUBSTITUTED CARBONYL OR NITRILE COMPOUNDS**

(71) Applicant: Universität Wien, 1010 Vienna (AT)
(72) Inventor: MAULIDE,, Prof. Dr. Nuno, 1090 Wien (AT); KLOSE,, Immo, 1090 Wien (AT); KNITTL-FRANK,, Christian, 1090 Wien (AT)
(74) Representative: Vossius & Partner Patentanwälte Rechtsanwälte mbB

(57) **Abstract**

A method for the preparation of an alpha-substituted carbonyl compound or an alpha-substituted nitrile compound carrying an aryl or heteroaryl group as an alpha-substituent, said method comprising reacting
a sulfoxide compound S1 comprising a substituted aryl or heteroaryl group, wherein a sulfinyl moiety is attached to a carbon ring atom of an aromatic ring,
with a reactant selected from the following reactants A1, A2 and A3:
an alkyne compound A1, which is selected from an ynamide, an ynolether, a thioalkyne and an arylalkyne, in the presence of an acid selected from a Bronsted- and a Lewis acid; a carbonyl compound A2, which is selected from a tertiary carboxylic acid amide which carries at least one hydrogen atom attached to a carbon atom in alpha-position to its carbonyl group and an aryl ketone which carries at least one hydrogen atom attached to a carbon atom in alpha-position to its carbonyl group, in the presence of an electrophilic activator; and a nucleophile A3, which is selected from a silyl enolether, an α-silyl nitrile, an α-stannyl nitrile, and a β-ketoester derivative, in the presence of an electrophilic activator;
to yield an alpha-substituted carbonyl compound or nitrile compound wherein a carbon atom in alpha-position to the carbonyl group or the nitrile group, respectively, forms a bond with the carbon ring atom of the aromatic ring to which the sulfinyl moiety had been attached in the sulfoxide compound S1.

## Description

The present invention relates to a method for the preparation of carbonyl compounds or nitrile compounds which carry an aryl substituent or a heteroaryl substituent in α-position (alpha-position) to their carbonyl or nitrile functionality.

The α-arylated carbonyl is a far spread structural motif of high importance in organic chemistry. Numerous natural products as well as drugs contain this scaffold. Many of these α-arylated carbonyl compounds can be synthesised using transition metal-catalysed (Pd and Ni) reactions, which are amongst the most powerful tools of organic chemistry to construct carbon-carbon bonds (Fabio Bellina and Renzo Rossi, Chem. Rev. 2010, 110, 1082-1146 and references therein; Fox, J. M.; Huang, X.; Chieffi, A.; Buchwald, S. L. J. Am. Chem. Soc. 2000, 122, 1360; Lee, S.; Beare, N. A.; Hartwig, J. F. J. Am. Chem. Soc. 2001, 123, 8410; Jorgensen, M.; Lee, S.; Liu, X.; Wolkowski, J. P.; Hartwig, J. F. J. Am. Chem. Soc. 2002, 124, 12557; Viciu, M. S.; Germaneau, R. F.; Nolan, S. P. Org. Lett. 2002, 4, 4053; Viciu, M. S.; Germaneau, R. F.; You, J.; Verkade, J. G. J. Org. Chem. 2003, 68, 8003; Hama, T.; Culkin, D. A.; Hartwig, J. F. J. Am. Chem. Soc. 2006, 128, 4976; Grasa, G. A.; Colacot, T. J. Org. Lett. 2007, 9, 5489; Alexander T. Wolters, Valentin Hornillos, Dorus Heijnen, Massimo Giannerini, Ben L. Feringa, ACS Catal. 2016, 6, 2622-2625). However, due to limitations in these methods several scaffolds still present a synthetic challenge. These limitations can be encountered with sterically hindered systems, highly electron rich systems - due to a slow oxidative addition - or substrates bearing several halide substituents, resulting chemoselectivity issues (Rylan J. Lundgren and Mark Stradiotto, Chem. Eur. J. 2012, 18, 9758-9769). Moreover, the high cost of palladium and its ligands as well as the risk of contamination in pharmaceutical applications are further critical factors. Especially α-carbonyl couplings require strongly basic conditions, which usually conflict with the preparation of enantioenriched compounds with tertiary stereocenters and, often times, necessitate the use of preformed silyl enolethers (Kuwajima, I.; Urabe, H. J. Am. Chem. Soc. 1982, 104, 6831-6833; Hamann, B. C.; Hartwig, J. F. J. Am. Chem. Soc. 1997, 119, 12382-12383; Su, W.; Raders, S.; Verkade, J. G.; Liao, X.; Hartwig, J. F. Angew. Chem. Int. Ed. 2006, 45, 5852-5855). Alternatively, the polarity reversed enantioselective arylation of racemic α-halo carbonyls using Nickel or iron catalysts has been developed (Sha Lou, Gregory C. Fu, J. Am. Chem. Soc. 2010, 132, 1264-1266; Pamela M. Lundin, Gregory C. Fu, J. Am. Chem. Soc. 2010, 132, 11027-11029; for an iron catalysed process, see: Takahiro Iwamoto, Chiemi Okuzono, Laksmikanta Adak, Masayoshi Jinc, Masaharu Nakamura, Chem. Commun. 2019, 55, 1128-1131). Yet, electron-rich and sterically encumbered systems remain unsolved.

Another approach, reported by Guiry and co-workers, uses a Pd-catalysed decarboxylation to convert ketoesters to enantioenriched tertiary α-arylated cyclic ketones (Cian Kingston, Jinju James, Patrick J. Guiry, J. Org. Chem. 2019, 84, 473-485). While this method allows the arylation of cyclic systems with electron rich and hindered arenes, synthesis of the substrates requires precarious organolead chemistry for the key C-C bond formation.

Especially in recent years, many patent applications that highlight the herbicidal potency of a-aryl carbonyls bearing electron-rich *ortho,ortho*-disubstituted aromatics were filed (e.g. WO 2019158666; JP 2019112310; WO 2017178314; WO 2016098899; WO 2015197468; WO 2015040114).

Thus, a new method allowing facile access to this motif is highly desired. For recent examples for alternative methods to obtain o,o'-disubstitutes α-aryl compunds, see a) Zhonglei Wang, Liansuo Zu, Chem. Commun. 2019, 55, 5171; b) Qianwen Gao, Ze-Shui Liu, Yu Hua, Lisha Li, Hong-Gang Cheng, Hengjiang Cong, Qianghui Zhou, Chem. Commun. 2019, 55, 8816; c) Yongtae Kim, Yun Soo Choi, Su Kyung Hong, Yong Sun Park, Org. Biomol. Chem. 2019, 17, 4554.

In this context, the present invention provides a metal-free cross coupling reaction using readily available sulfoxides to afford α-arylated carbonyls or nitriles. In the last decade, aryl sulfoxides were introduced as extremely versatile directing handles for metal-free, regioselective functionalisation of the *ortho*-position. For reviews on sulfoxide mediated rearrangements, see a) A.P. Pulis, D. J. Procter, Angew. Chem. Int. Ed. 2016, 55, 9842; b) Hideki Yorimitsu, Chem. Rec. 2017, 17, 1156-1167; c) Tomoyuki Yanagi, Keisuke Nogi, Hideki Yorimitsu, Tet. Lett. 2018, 59, 2951-2959; d) Daniel Kaiser, Immo Klose, Rik Oost, James Neuhaus, Nuno Maulide, Chemical Reviews 2019, 119, 8701-8780. Recent work demonstrated that the regioselectivity can be altered to functionalise the meta-position as well (Maryasin, B.; Kaldre, D.; Galaverna, R.; Klose, I.; Ruider, S.; Drescher, M.; Kählig, H.; Gonzalez, L.; Eberlin, M. N.; Jurberg, I. D.; Maulide, N. Chem. Sci. 2018, 9, 4124-4131). Moreover, a Pummerer variant that leads to ipso-displacement of the sulfoxide with oxygen has been described in the literature, see: a) Shuji Akai, Keisuke Kakiguchi, Yuka Nakamura, Ikumi Kuriwaki, Toshifumi Dohi, Shusaku Harada, Ozora Kubo, Nobuyoshi Morita, Yasuyuki Kita, Angew. Chem. Int. Ed. 2007, 46, 7458 -7461; b) S. Akai, T. Takeda, K. lio, Y. Yoshida, Y. Kita, J. Chem. Soc. Chem. Commun. 1995, 1013-1014; c) Shuji Akai, Kiyosei lio, Yoshifumi Takeda, Hiroshi Ueno, Kayoko Yokogawa Yasuyuki Kita, J. Chem. Soc. Chem. Comm. 1995, 2319-2320; d) Shuji Akai, Yoshifumi Takeda, Kiyosei lio, Kenji Takahashi, Nobuhisa Fukuda, Yasuyuki Kita, J. Org. Chem. 1997, 21, 5526-5536.

Herein, a new approach is presented to form a C-C bond at the ipso-position to provide an α-substituted carbonyl or nitrile compound.

The method of the present invention allows an alpha-substituted carbonyl or nitrile compound, which carries an aryl substituent or a heteroaryl substituent in alpha-position to the carbonyl group or the nitrile group, to be conveniently provided without the need for, e.g. the presence of a transition metal or a transition metal compound which acts as a catalyst during the reaction, and/or without the need for strongly basic conditions, which may be detrimental for the stability of certain starting compounds or of certain desired end products. Moreover, the method is suitable to prepare congested arylated or heteroarylated compounds, i.e. compounds where the accessibility of the alpha-carbon atom is restricted due to the presence of further substituents, in particular further bulky substituents.

The present invention is thus summarized in the following items.
1. A method for the preparation of an alpha-substituted carbonyl compound or an alpha-substituted nitrile compound carrying an aryl or heteroaryl group as an alpha-substituent, said method comprising reacting
   (i) a sulfoxide compound S1 comprising a substituted aryl or heteroaryl group, wherein a sulfinyl moiety is attached to a carbon ring atom of an aromatic ring,
      with a reactant selected from the following reactants A1, A2 and A3:
      (ii-1) an alkyne compound A1, which is selected from an ynamide A1a, an ynolether A1b, a thioalkyne A1c and an arylalkyne A1d, in the presence of an acid selected from a Brønsted- and a Lewis acid;
      (ii-2) a carbonyl compound A2, which is selected from a tertiary carboxylic acid amide A2a which carries at least one hydrogen atom attached to a carbon atom in alpha-position to its carbonyl group and an aryl ketone A2b which carries at least one hydrogen atom attached to a carbon atom in alpha-position to its carbonyl group, in the presence of an electrophilic activator; and
      (ii-3) a nucleophile A3, which is selected from a silyl enolether A3a, an α-silyl nitrile A3b, an α-stannyl nitrile A3c, and a β-ketoester derivative A3d, in the presence of an electrophilic activator;
         to yield an alpha-substituted carbonyl compound or nitrile compound wherein a carbon atom in alpha-position to the carbonyl group or the nitrile group, respectively, forms a bond with the carbon ring atom of the aromatic ring to which the sulfinyl moiety had been attached in the sulfoxide compound S1.
2. The method of item 1, wherein the aryl group or heteroaryl group comprised by S1 contains a monocyclic 5- or 6-membered aromatic ring, and a sulfinyl moiety attached to a carbon ring atom of the aromatic ring, or a 9- or 10-membered bicyclic aromatic ring system, and a sulfinyl moiety attached to a carbon ring atom of an aromatic ring forming part of the ring system.
3. The method of item 1 or 2, wherein the sulfinyl group is provided by a substituent -S(O)-R^{1S} which is attached to a carbon ring atom of an aromatic ring, wherein R^{1S} is a hydrocarbyl group, preferably a C1-C6 alkyl group.
4. The method of any of items 1 to 3, wherein the sulfoxide compound is a sulfoxide compound wherein the sulfinyl moiety is attached to a carbon ring atom of a 6-membered carbocyclic aromatic ring which may be part of a fused aromatic ring system, and the 6-membered carbocyclic aromatic ring carries at least two further substituents in addition to the sulfinyl moiety, namely:
   (i) an electron donating substituent selected from -OH, -OR^{1D}, -NH₂, -NHR^{2D} and -NR^{2D}R^{3D} in *ortho* or *para*-position relative to the sulfinyl moiety, wherein R^{1D}, R^{2D} and R^{3D} are independently a hydrocarbyl group;
      and
   (ii) a substituent in *ortho*-position relative to the sulfinyl moiety, which may be linked with a further substituent carried by the 6-membered aromatic ring to form a ring which is fused to the 6-membered aromatic ring.
5. The method of item 4, wherein the 6-membered carbocyclic aromatic ring of the preferred sulfoxide compound carries at least two further substituents which are both an electron donating substituent selected from -OH, -OR^{1D}, -NH₂, -NHR^{2D} and -NR^{2D}R^{3D}, wherein R^{1D}, R^{2D} and R^{3D} are independently a hydrocarbyl group, one of them being in *ortho-*position and the other one being in *ortho* or *para*-position relative to the sulfinyl moiety.
6. The method of any of items 1 to 3, wherein the sulfoxide compound is a sulfoxide compound wherein the sulfinyl moiety is attached to a carbon ring atom of a 5-membered heterocyclic aromatic ring which may be part of a fused aromatic ring system, and wherein the 5-membered carboxylic aromatic ring carries at least one further substituent in *ortho-*position relative to the sulfinyl moiety, which may be linked with a further substituent on the 5-membered aromatic ring to form a ring which is fused to the 5-membered aromatic ring.
7. The method of item 6, wherein the 5-membered heterocyclic aromatic ring either
   (i) carries at least two substituents, which are attached to carbon ring atoms in *ortho-*position to the sulfinyl moiety and which may be linked with a further substituent on the 5-membered aromatic ring to form a ring which is fused to the 5-membered aromatic ring; or
   (ii) carries one substituent in *ortho*-position to the sulfinyl moiety, which may be linked with a further substituent on the 5-membered aromatic ring to form a ring which is fused to the 5-membered aromatic ring, and the 5-membered aromatic ring additionally has a heteroatom as a ring member adjacent to the carbon atom to which the sulfinyl moiety is attached.
8. The method of any of items 1 to 7, wherein the sulfoxide compound S1 is reacted with an alkyne compound A1 selected from:
   a) an ynamide represented by formula (2) or (3) 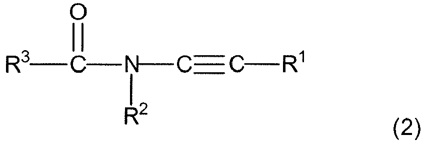 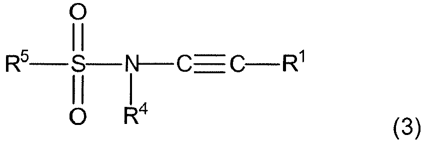 wherein
      R¹ is selected from hydrogen, an organyl group, and halogen; R² and R⁴ are an organyl group; R³ is selected from an organyl group and an oxyorganyl group; R⁵ is an organyl group; and R² and R³ may be linked to form a ring together with the atoms to which they are attached;
   b) an ynolether or a thioalkyne represented by formula (4) or (5)

      R⁶-O-C≡C-R¹ (4)

      R⁷-S-C≡C-R¹ (5)

      wherein
      R¹ is selected from hydrogen, an organyl group, an oxyorganyl group, and halogen; and R⁶ and R⁷ are an organyl group; and
   c) an arylalkyne represented by formula (6)

      Ar¹C≡C-R¹ (6)

      wherein
      R¹ is selected from hydrogen, an organyl group and halogen; and Ar¹ is an optionally substituted aryl group.
9. The method of any of items 1 to 8, wherein the sulfoxide compound S1 is reacted with the alkyne compound A1, and the reaction is carried out in the presence of a Bronsted acid having a pKa of less than -2.5.
10. The method of any of items 1 to 7, wherein the sulfoxide compound S1 is reacted with a carbonyl compound A2 selected from:
   a) a tertiary carboxylic acid amide represented by formula (7) 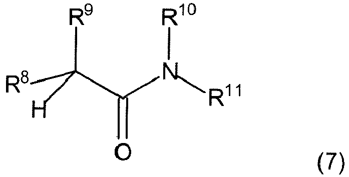 wherein
      R⁸ and R⁹ are independently selected from hydrogen and an organyl group; and R¹⁰ and R¹¹ are independently selected from hydrogen and an organyl group; and
   b) an aryl ketone represented by formula (8) 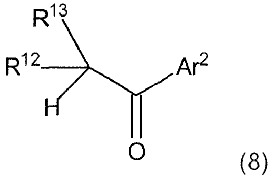 wherein
      Ar² is an optionally substituted aryl group, e.g. an optionally substituted phenyl or naphthyl group; and R¹² and R¹³ are independently selected from hydrogen and an organyl group.
11. The method of any of items 1 to 7 and 10, wherein the sulfoxide compound S1 is reacted with a carbonyl compound A2 in the presence of trifluoromethanesulfonic acid anhydride as an electrophilic activator.
12. The method of any of items 1 to 7, wherein the sulfoxide compound S1 is reacted with a nucleophile A3 selected from:
   a) a silyl enolether represented by formula (9) 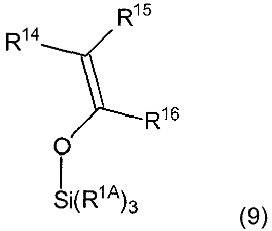 wherein
      R^{1A}, independently for each occurrence, is a hydrocarbyl group, and R¹⁴ to R¹⁶ are independently selected from hydrogen and an organyl group;
   b) an α-silyl nitrile or an α-stannyl nitrile represented by formula (10) 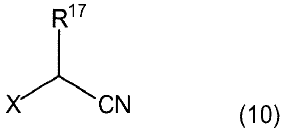 wherein
      X is selected from -Si(R^{2A})₃ and -Sn(R^{3A})₃, R^{2A} and R^{3A} are, independently for each occurrence, a hydrocarbyl group, and R¹⁷ is selected from hydrogen, and an organyl group; and
   c) a β-ketoester derivative represented by formula (11) 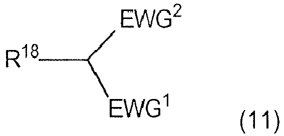 wherein
      EWG¹ and EWG² are the same or different, and are selected from -C(O)R^{4A}, -C(O)OR^{5A}, -C(O)NR^{6A}R^{7A}, and CN, with R^{4A}, R^{5A}, R^{6A} and R^{7A} being independently selected from hydrogen and a hydrocarbyl group; and R¹⁸ is selected from hydrogen and an organyl group.
13. The method of any of items 1 to 7 and 12, wherein the sulfoxide compound S1 is reacted with the nucleophile A3 in the presence of an electrophilic activator selected from trifluoromethanesulfonic acid anhydride, trifluoroacetic acid anhydride, toluenesulfonic anhydride, methanesulfonic anhydride, and TfOC(O)CF₃.
14. The method of any of items 1 to 13, wherein the sulfoxide compound S1 is reacted with the alkyne compound A1, the carbonyl compound A2 or the nucleophile A3 in the further presence of a soft nucleophile as an additive.
15. The method of item 14, wherein the soft nucleophile is selected from octanethiol, phenylthiol, 2-methyl-2-butene, and 2,3-dimethylbut-2-ene.

In line with the above, the method of the present invention may be used to prepare an alpha-substituted (or α-substituted) carbonyl compound which carries an aryl substituent or a heteroaryl substituent in alpha-position to the carbonyl group. In order to facilitate the following discussion, such a compound may be referred to herein as "alpha-substituted carbonyl compound". As will be appreciated by the skilled reader, a carbonyl compound is a compound which comprises a moiety -C(O)-. As will be further understood, the alpha-substituted carbonyl compound is a carbonyl compound wherein a carbon atom which is in alpha-position to the -C(O)- moiety, forms a bond (a covalent bond) with a ring atom of the aryl substituent or of the heteroaryl substituent. The carbon atom in alpha-position to the -C(O)- moiety is a carbon atom adjacent to the carbonyl moiety which is attached via a bond (a covalent bond) to the carbon atom of the -C(O)- moiety. More specifically, in the case of the alpha-substituted carbonyl compound provided by the method in accordance with the method of the present invention, the bond is formed between a carbon atom which is in alpha-position to the -C(O)- moiety, and the carbon ring atom of the aryl substituent or of the heteroaryl substituent to which the sulfinyl moiety had been attached in the sulfoxide compound S1 used as a starting compound.

Alternatively, the method of the present invention may be used to prepare an alpha-substituted (or α-substituted) nitrile compound which carries an aryl substituent or a heteroaryl substituent in alpha-position to the nitrile group. In order to facilitate the following discussion, such a compound may be referred to herein as "alpha-substituted nitrile compound". As will be appreciated by the skilled reader, a nitrile compound is a compound which comprises a nitrile group -CN. As will be understood, the alpha-substituted nitrile compound is a nitrile compound wherein a carbon atom which is in alpha-position to the nitrile group forms a bond (a covalent bond) with a ring atom of the aryl substituent or of the heteroaryl substituent. The carbon atom in alpha-position to the -CN group is a carbon atom adjacent to the -CN group which is attached via a bond (a covalent bond) to the carbon atom of the nitrile group. More specifically, in the case of the alpha-substituted nitrile compound provided by the method in accordance with the method of the present invention, the bond is formed between a carbon atom which is in alpha-position to the nitrile group and the carbon ring atom of the aryl substituent or of the heteroaryl substituent to which the sulfinyl moiety had been attached in the sulfoxide compound S1 used as a starting compound.

As will be appreciated by the skilled reader, the type of reaction product provided by the method of the present invention, i.e. whether an alpha-substitued carbonyl compound or an alpha-substituted nitrile compound is provided, is determined by the type of reactant A1, A2 or A3 used in the method. If the sulfoxide compound S1 is reacted with an alkyne compound A1, or with a carbonyl compound A2, the method of the invention yields an alpha-substituted carbonyl compound. If the sulfoxide compound is reacted with a nucleophile A3 which is a silyl enolether A3a or a β-ketoester derivative A3d comprising a carbonyl moiety and no nitrile group, the method of the invention equally yields an alpha-substituted carbonyl compound. If the sulfoxide compound is reacted with a nucleophile A3 which is an α-silyl nitrile A3b, an α-stannyl nitrile A3c, or a β-ketoester derivative A3d comprising a nitrile group and no carbonyl group, the method of the invention yields an alpha-substituted nitrile compound. If the reactant A3d is a β-ketoester derivative containing a nitrile group and a carbonyl moiety, the method of the invention can provide an alpha-substituted compound wherein the aryl or the heteroaryl group is in alpha-position to both a nitrile group and a carbonyl moiety. It will be understood that the products which are provided by the method of the present invention can be converted by known means, e.g. the nitrile group of a nitrile compound can be hydrolyzed to yield an amide or a salt of a carboxylic acid.

Thus, in line with one preferred embodiment, the invention provides a method for the preparation of an alpha-substituted carbonyl compound carrying an aryl or heteroaryl group as an alpha-substituent, said method comprising reacting
(i) a sulfoxide compound S1 comprising a substituted aryl or heteroaryl group, wherein a sulfinyl moiety is attached to a carbon ring atom of an aromatic ring,
   with a reactant selected from the following reactants A1, A2 and A3:
   (ii-1) an alkyne compound A1, which is selected from an ynamide A1a, an ynolether A1b, a thioalkyne A1c and an arylalkyne A1d, in the presence of an acid selected from a Bronsted- and a Lewis acid;
   (ii-2) a carbonyl compound A2, which is selected from a tertiary carboxylic acid amide A2a which carries at least one hydrogen atom attached to a carbon atom in alpha-position to its carbonyl group and an aryl ketone A2b which carries at least one hydrogen atom attached to a carbon atom in alpha-position to its carbonyl group, in the presence of an electrophilic activator; and
   (ii-3) a nucleophile A3, which is selected from a silyl enolether A3a and a β-ketoester derivative A3d comprising a carbonyl group, in the presence of an electrophilic activator; to yield an alpha-substituted carbonyl compound wherein a carbon atom in alpha-position to the carbonyl group forms a bond with the carbon ring atom of the aromatic ring to which the sulfinyl moiety had been attached in the sulfoxide compound S1.

In line with another preferred embodiment, the invention provides a method for the preparation of an alpha-substituted nitrile compound carrying an aryl or heteroaryl group as an alpha-substituent, said method comprising reacting
(i) a sulfoxide compound S1 comprising a substituted aryl or heteroaryl group, wherein a sulfinyl moiety is attached to a carbon ring atom of an aromatic ring, with
(ii-3) a nucleophile A3, which is selected from an α-silyl nitrile A3b, an α-stannyl nitrile A3c, and a β-ketoester derivative A3d comprising a nitrile group, in the presence of an electrophilic activator;
   to yield an alpha-substituted nitrile compound wherein a carbon atom in alpha-position to the nitrile group forms a bond with the carbon ring atom of the aromatic ring to which the sulfinyl moiety had been attached in the sulfoxide compound S1.

Unless indicated otherwise, the following explanation applies both to the variant of the method in accordance with the invention which provides an alpha-substituted carbonyl compound and to the variant which provides an alpha-substituted nitrile compound.

The sulfoxide compound S1 (also referred to as "S1" herein) is a compound comprising a substituted aryl or heteroaryl group. The aryl or heteroaryl group may be collectively referred to as "aromatic group" herein. The aryl or heteroaryl group comprises an aromatic ring, and a sulfinyl moiety -S(O)- is attached to a carbon ring atom of an aromatic ring. In line with common understanding, the ring atom is an atom which acts as a ring member of the aromatic ring. The aromatic ring may be a carbocyclic aromatic ring, i.e. a ring formed exclusively by carbon atoms as ring atoms, or a heterocyclic aromatic ring, i.e. a ring formed by carbon atoms and one or more, e.g. one, two or three, heteroatoms. Suitable heteroatoms are, for example, selected from N, S and O. The aromatic ring may be fused with one or more further rings, and may e.g. be part of a fused aromatic ring system. It will be understood that the aryl or heteroaryl group may carry further substituents in addition to the substituent which comprises the sulfinyl moiety.

The aryl group or heteroaryl group comprised by S1 preferably contains a monocyclic 5- or 6-membered aromatic ring, and a sulfinyl moiety attached to a carbon ring atom of the aromatic ring, or a 9- or 10-membered bicyclic aromatic ring system, and a sulfinyl moiety attached to a carbon ring atom of an aromatic ring forming part of the ring system. The monocyclic 6-membered aromatic ring is preferably a benzene ring. The monocyclic 5-membered aromatic ring is preferably a heterocyclic aromatic ring comprising one or more, e.g. one, two or three, heteroatoms as ring atoms. Suitable heteroatoms are, for example, selected from N, S and O. The 9-membered bicyclic aromatic ring system is preferably a heterocyclic aromatic ring system comprising one or more, e.g. one, two or three, heteroatoms as ring atoms. Suitable heteroatoms are, for example, selected from N, S and O. The 10-membered bicyclic aromatic ring system is preferably a naphthalene ring system.

The sulfoxide compound comprises a sulfinyl moiety -S(O)- attached to a carbon ring atom of an aromatic ring, i.e. one of the bonds of the sulfinyl moiety is formed directly with the carbon ring atom. Preferably, the sulfinyl group is provided by a substituent -S(O)-R^{1S} which is attached to a carbon ring atom of an aromatic ring, wherein R^{1S} is a hydrocarbyl group, such as an alkyl group or aryl group, preferably a C1-C6 alkyl group, and more preferably a methyl group. As will be understood from the above, the open bond of the substituent -S(O)-R^{1S} is attached to the carbon ring atom of the aromatic ring.

The reaction of the sulfoxide compound S1 in the method of the present invention proceeds particularly efficiently if the aromatic ring to which the sulfinyl group is attached is an electron-rich aromatic ring and/or if the aromatic ring to which the sulfinyl group is attached carries further substituents, preferably sterically demanding substituents, in one or both ortho-positions relative to the sulfinyl group.

In line with the above, preferred variants of the sulfoxide compound S1 are the following a) and b).
a) A sulfoxide compound wherein the sulfinyl moiety, preferably the substituent -S(O)-R^{1S} as defined above, is attached to a carbon ring atom of a 6-membered carbocyclic aromatic ring which may be part of a fused aromatic ring system, e.g. of a 10-membered bicyclic aromatic ring system. Specific examples of such a sulfoxide compound are a sulfoxide compound comprising a substituted phenyl group, and a sulfoxide compound comprising a substituted naphthyl group. In addition to the sulfinyl moiety, the 6-membered carbocyclic aromatic ring of this preferred sulfoxide compound carries at least two further substituents, namely:
   (i) an electron donating substituent selected from -OH, -OR^{1D}, -NH₂, -NHR^{2D} and -NR^{2D}R^{3D} in *ortho* or *para*-position, more preferably in *ortho*-position, relative to the sulfinyl moiety, wherein R^{1D}, R^{2D} and R^{3D} are independently a hydrocarbyl group, such as an alkyl group, a haloalkyl group, an alkenyl group or an aralkyl group, and preferably an alkyl group; and
   (ii) a substituent (i.e. an atom or group which is not H) in *ortho*-position relative to the sulfinyl moiety. Examples of such a substituent include an electron donating substituent as defined above, halogen (e.g. chloro or bromo), alkyl (e.g. C1-C6 alkyl), haloalkyl (e.g. C1-C6 alkyl carrying one halogen atom), and alkenyl (e.g. C2-C6 alkenyl). Such a substituent in *ortho*-position may be linked with a further substituent carried by the 6-membered aromatic ring to form a ring which is fused to the 6-membered aromatic ring.
   It is still more preferred within variant a) that the 6-membered carbocyclic aromatic ring of the preferred sulfoxide compound carries at least two further substituents which are both an electron donating substituent as defined above, one of them being in *ortho*-position and the other one being in *ortho* or *para*-position relative to the sulfinyl moiety.
b) A sulfoxide compound wherein the sulfinyl moiety, preferably the substituent -S(O)-R^{1S} as defined above, is attached to a carbon ring atom of a 5-membered heterocyclic aromatic ring which may be part of a fused aromatic ring system, e.g. of a 9-membered bicyclic aromatic ring system. Specific examples of such a sulfoxide compound are a sulfoxide compound comprising a substituted furanyl group, a substituted thiophenyl group, a substituted pyrrolyl group or a substituted indolyl group. In addition to the sulfinyl moiety, the 5-membered carboxylic aromatic ring carries at least one further substituent (i.e. an atom or group which is not H) in *ortho*-position relative to the sulfinyl moiety. Examples of such a substituent include an electron donating substituent selected from -OH, -OR^{1D}, -NH₂, -NHR^{2D} and -NR^{2D}R^{3D}, halogen (e.g. chloro or bromo), alkyl (e.g. C1-C6 alkyl), haloalkyl (e.g. C1-C6 alkyl carrying one halogen atom), and alkenyl (e.g. C2-C6 alkenyl). Such a substituent in *ortho*-position may be linked with a further substituent on the 5-membered aromatic ring to form a ring which is fused to the 5-membered aromatic ring. R^{1D}, R^{2D} and R^{3D} are independently a hydrocarbyl group, such as an alkyl group, a haloalkyl group, an alkenyl group or an aralkyl group, and preferably an alkyl group.

It is still more preferred within variant b) that the 5-membered heterocyclic aromatic ring either
(i) carries at least two substituents (i.e. atoms or groups which are not H) which are attached to carbon ring atoms in *ortho*-position to the sulfinyl moiety, with examples of the substituent including an electron donating substituent as defined above, halogen (e.g. chloro or bromo), alkyl (e.g. C1-C6 alkyl), haloalkyl (e.g. C1-C6 alkyl carrying one halogen atom), and alkenyl (e.g. C2-C6 alkenyl), and the substituent may be linked with a further substituent on the 5-membered aromatic ring to form a ring which is fused to the 5-membered aromatic ring; or
(ii) carries one substituent in *ortho*-position to the sulfinyl moiety, and additionally has a heteroatom (e.g. N, S or O) as a ring member adjacent to the carbon atom to which the sulfinyl moiety is attached, with examples of the substituent including an electron donating substituent as defined above, halogen (e.g. chloro, bromo), alkyl (e.g. C1-C6 alkyl), haloalkyl (e.g. C1-C6 alkyl carrying one halogen atom), and alkenyl (e.g. C2-C6 alkenyl), and the substituent may be linked with a further substituent on the 5-membered aromatic ring to form a ring which is fused to the 5-membered aromatic ring.

In line with the above, a preferred sulfoxide compound S1 for use in the method of the present invention can be illustrated by the following formula: 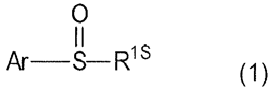 wherein Ar is an aryl or heteroaryl group as defined above, which may carry further substituents in addition to the group -S(O)-R^{1S}, and R^{1S} is as defined above, including any preferred embodiments. With regard to the preferred structures of the aromatic ring or the aromatic ring system comprised by the aryl or heteroaryl group, and with regard to preferred further substituents carried by the aromatic ring, the information provided above with regard to preferred embodiments of the sulfoxide compound S1 equally applies.

In accordance with a first embodiment of the method in accordance with the invention, an alpha-substituted carbonyl compound carrying an aryl or heteroaryl group as an alpha-substituent is provided by reacting the sulfoxide compound S1 (including its preferred variants discussed above) with an alkyne compound A1, which is selected from an ynamide A1a, an ynolether A1b, a thioalkyne A1c and an arylalkyne A1d, in the presence of an acid selected from a Bronsted- and a Lewis acid.

The ynamide A1a is an alkyne compound which comprises a C-C triple bond, and a carbonyl moiety -C(O)- or a sulfonyl moiety -S(O)₂- linked via a nitrogen atom to a carbon atom involved in the C-C triple bond.

The nitrogen atom which forms part of the amide group of the ynamide and which links the carbonyl moiety -C(O)- or sulfonyl moiety -S(O)₂- to a carbon atom involved in the C-C triple bond, is substituted, i.e. it does not carry a hydrogen atom.

Preferred structures of the ynamide can be illustrated by the following formulae: 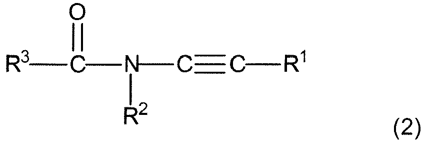 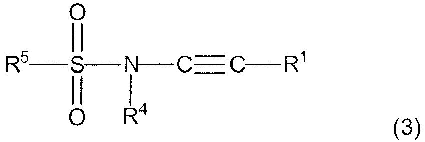 wherein
R¹ is selected from hydrogen, an organyl group, an oxyorganyl group, -OH, and halogen; R² and R⁴ are an organyl group, preferably a hydrocarbyl group, such as an alkyl group, an aryl group, or an aralkyl group; R³ is selected from an organyl group and an oxyorganyl group, preferably from an alkyl group and an oxyalkyl group; R⁵ is an organyl group, preferably an alkyl group; and R² and R³ may be linked to form a ring together with the atoms to which they are attached, e.g. a lactame ring or a cyclic carbamate, such as a 1,3-oxazolidin-2-one ring.

As used herein, the term organyl group refers to an organic substituent group having one free valency at a carbon atom. An example is a hydrocarbyl group, e.g. an alkyl group, a cylcloalkyl group, an aryl group or an aralkyl group. The term oxyorganyl group refers to group having a free valency at an oxygen atom, and an organyl group attached to a second valency of the oxygen atom, i.e. a group -O-organyl. An example of the organyl moiety in the oxyorganyl group is a hydrocarbyl group, e.g. e.g. an alkyl group, a cylcloalkyl group, an aryl group or an aralkyl group.

The reaction of the preferred sulfoxide of formula (1) with the preferred ynamide of formula (2) or (3) to yield an alpha-substituted carbonyl compound in accordance with the method of the present invention can thus be illustrated by the following reaction schemes: 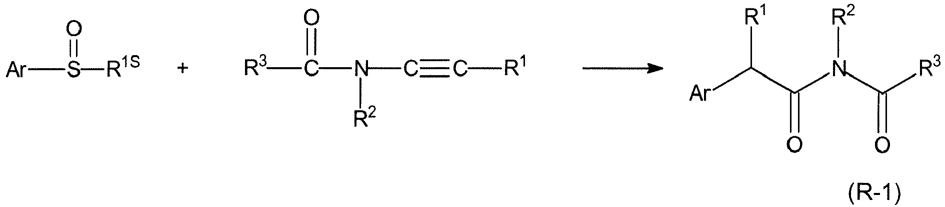 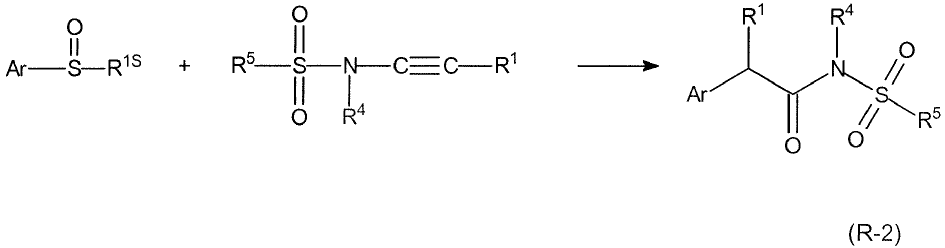 wherein the variables Ar, R^{1S} and R¹ to R⁵ are as defined above.

The ynolether A1b is an alkyne compound which comprises a C-C triple bond and an organyl group linked via an ether bond -O- to a carbon atom involved in the C-C triple bond. Similarly, the thioalkyne A1c is an alkyne compound which comprises a C-C triple bond and an organyl group linked via a thioether bond -S- to a carbon atom involved in the C-C triple bond.

Preferred structures of the ynolether and the thioalkyne can be illustrated by the following formulae:

R⁶-O-C≡C-R¹ (4)

R⁷-S-C≡C-R¹ (5)

wherein
R¹ is selected from hydrogen, an organyl group, an oxyorganyl group and halogen; and R⁶ and R⁷ are an organyl group, preferably a hydrocarbyl group, and are more preferably an alkyl group.

The reaction of the preferred sulfoxide of formula (1) with the preferred ynolether of formula (4) or the preferred thioalkyne of formula (5) to yield an alpha-substituted carbonyl compound in accordance with the method of the present invention can thus be illustrated by the following reaction schemes: 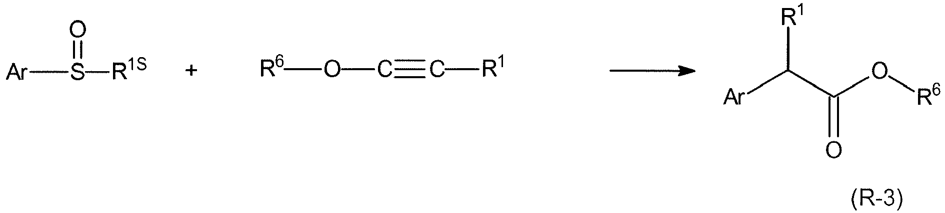 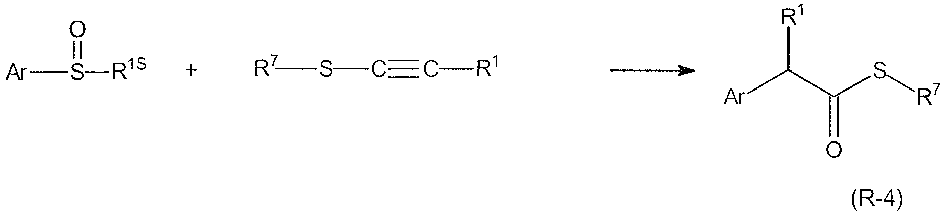 wherein the variables Ar, R^{1S}, R¹, R⁶ and R⁷ are as defined above.

The arylalkyne A1d is an alkyne compound which comprises a C-C triple bond and an optionally substituted aryl group attached to a carbon atom involved in the C-C triple bond.

A preferred structure of the arylalkyne can be illustrated by the following formula:

Ar¹-C≡C-R¹ (6)

wherein
R¹ is selected from hydrogen, an organyl group and halogen; and Ar¹ is an optionally substituted aryl group, e.g. an optionally substituted phenyl or naphthyl group. Preferably, Ar¹ is an electron rich aryl group, i.e. an aryl group with an electron rich aromatic ring or ring system. Thus, it is particularly preferred that the aryl group comprises one or more, such as one, two or three, substituents selected from alkyl, aryl, -OH, -OR^{1D}, -NH₂, -NHR^{2D}, -NR^{2D}R^{3D} in *ortho* or para-position to the alkyne, wherein R^{1D}, R^{2D} and R^{3D} are independently a hydrocarbyl group, such as an alkyl group, a haloalkyl group, an alkenyl group or an aralkyl group, and are preferably an alkyl group.

The reaction of the preferred sulfoxide of formula (1) with the preferred arylalkyne of formula (6) to yield an alpha-substituted carbonyl compound in accordance with the method of the present invention can thus be illustrated by the following reaction scheme: 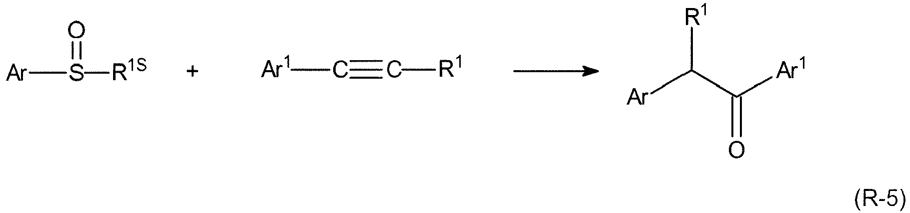 wherein the variables Ar, R^{1S}, R¹ and Ar¹ are as defined above.

The reaction of the sulfoxide compound S1 (and its preferred variants discussed above) with the alkyne compound A1 (and its preferred variants discussed above) is carried out in the presence of an acid selected from a Bronsted and a Lewis acid. Preferably, it is carried out in the presence of a Bronsted acid having a pKa (as determined in water at 20°C) of less than -2.5.

Examples of suitable Bronsted acids include 4-nitrobenzene sulfonic acid, trifluoromethanesulfonic acid (triflic acid), bis(trifluoromethane)sulfonamide (bistriflimide, Tf₂NH) and 1,1,2,2,3,3-hexafluoropropane-1,3-disulfonimide. Examples of suitable Lewis acids include Triphenylphosphine gold chloride, IPrAuNTf₂, [JohnPhosAuNCMe][SbF₆], AgOTf, AgBF₄, AgPF₆, Silver triflimide, Fe(OTf)₂, In(OTf)₃, Yb(OTf)₃, Sc(OTf)₃, Zn(OTf)₂, and BF₃*OEt₂. As noted above, the Bronsted acids are preferred, and among them trifluoromethanesulfonic acid and bis(trifluoromethane)sulfonamide are further preferred.

Preferably, the reaction of the sulfoxide compound S1 (and its preferred variants discussed above) with the alkyne compound A1 (and its preferred variants discussed above) is carried out in the further presence of a soft nucleophile as an additive which assists in obtaining the product at a high yield and in reducing the byproduct formation. As exemplary classes of compounds which can be used as soft nucleophiles, reference can be made to thiol compounds, such as alkylthiols or arylthiols, and to alkenes, arenes and heteroarenes with a N-Parameter higher than 3.5 according to the Mayr's Database of Reactivity Parameters (e.g. https://www.cup.Imu.de/oc/mayr/reaktionsdatenbank). Exemplary compounds which can be suitably used as such an additive (a soft nucleophile) include octanethiol, phenylthiol, 2-methyl-2-butene, and 2,3-dimethylbut-2-ene.

For the reaction, the sulfoxide compound S1 (and its preferred variants discussed above) and the alkyne compound A1 (and its preferred variants discussed above) are typically used in a molar ratio ranging from 2/1 to 1/2, and preferably at a molar ratio of 1/1.0 - 1.5.

The acid is preferably present during the reaction in an amount of 1 to 150 mol%, more preferably 5 to 50 mol%, and still more preferably 5 to 30 mol%, based on the molar amount of the sulfoxide compound S1 as 100 mol%.

The soft nucleophile as an additive, if present during the reaction, is preferably used in an amount of 100 to 150 mol%, based on the molar amount of the sulfoxide compound S1 as 100 mol%.

The reaction can be conveniently accomplished by contacting the sulfoxide compound S1 (and its preferred variants discussed above), the alkyne compound A1 (and its preferred variants discussed above) the acid and optionally the soft nucleophile in a suitable solvent, preferably an aprotic solvent, such as CH₂Cl₂, nitromethane or toluene. The reaction temperature can be suitable adjusted, typically the reaction is carried out at a temperature in the range of -10 °C to 50 °C, preferably in the range of 0 °C to 25 °C. Once the reaction has been allowed to proceed to a sufficient extent, e.g. over 1 to 10 hours, the obtained product can be isolated and, if necessary, purified by conventional methods.

In accordance with a second embodiment of the method in accordance with the invention, an alpha-substituted carbonyl compound carrying an aryl or heteroaryl group as an alpha-substituent is provided by reacting the sulfoxide compound S1 (including its preferred variants discussed above) with a carbonyl compound A2, which is selected from a tertiary carboxylic acid amide A2a which carries at least one hydrogen atom attached to a carbon atom in alpha-position to its carbonyl group and an aryl ketone A2b which carries at least one hydrogen atom attached to a carbon atom in alpha-position to its carbonyl group, in the presence of an electrophilic activator.

A preferred structure of the tertiary carboxylic acid amide A2a can be illustrated by the following formula: 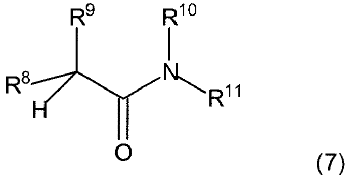 wherein
R⁸ and R⁹ are independently selected from hydrogen and an organyl group and R¹⁰ and R¹¹ are independently selected from hydrogen and an organyl group, preferably from hydrogen and a hydrocarbyl group, such as an alkyl group, an aryl group, or an aralkyl group.

The reaction of the preferred sulfoxide of formula (1) with the preferred tertiary carboxylic acid amide of formula (7) to yield an alpha-substituted carbonyl compound in accordance with the method of the present invention can thus be illustrated by the following reaction scheme: 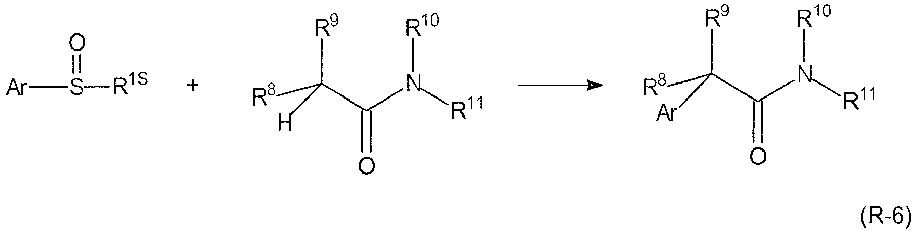 wherein the variables Ar, R^{1S} and R⁸ to R¹¹ are as defined above.

A preferred structure of the aryl ketone A2b can be illustrated by the following formula: 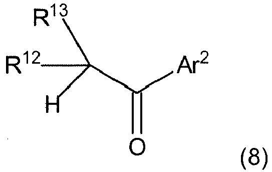 wherein
Ar² is an optionally substituted aryl group, e.g. an optionally substituted phenyl or naphthyl group; and R¹² and R¹³ are independently selected from hydrogen and an organyl group.

The reaction of the preferred sulfoxide of formula (1) with the preferred aryl ketone of formula (8) to yield an alpha-substituted carbonyl compound in accordance with the method of the present invention can thus be illustrated by the following reaction scheme: 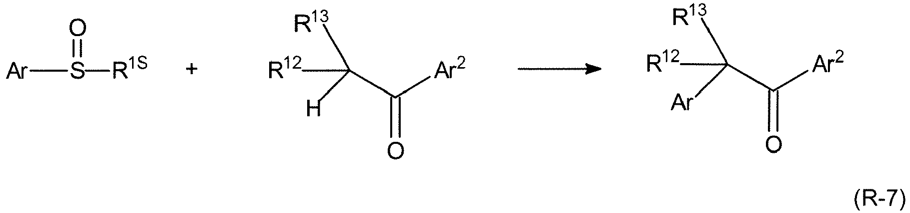 wherein the variables Ar, R^{1S}, Ar², R¹² and R¹³ are as defined above.

The reaction of the sulfoxide compound S1 (and its preferred variants discussed above) with the carbonyl compound A2 (and its preferred variants discussed above) is carried out in the presence of an electrophilic activator.

An example of a suitable electrophilic activator is trifluoromethanesulfonic acid anhydride (triflic anhydride, Tf₂O).

Preferably, the reaction of the sulfoxide compound S1 (and its preferred variants discussed above) with the carbonyl compound A2 (and its preferred variants discussed above) is carried out in the further presence of a soft nucleophile as an additive which assists in obtaining the product at a high yield and in reducing the byproduct formation. As exemplary classes of compounds which can be used as soft nucleophiles, reference can be made to thiol compounds, such as alkylthiols or arylthiols, and to alkenes, arenes and heteroarenes with a N-Parameter higher than 3.5 according to the Mayr's Database of Reactivity Parameters (e.g. https://www.cup.Imu.de/oc/mayr/reaktionsdatenbank). Exemplary compounds which can be suitably used as such an additive (a soft nucleophile) include octanethiol, phenylthiol, 2-methyl-2-butene, and 2,3-dimethylbut-2-ene.

For the reaction, the sulfoxide compound S1 (and its preferred variants discussed above) and the carbonyl compound A2 (and its preferred variants discussed above) are typically used in a molar ratio ranging from 2/1 to 1/2, and preferably at a molar ratio of about 1/1.0-1.5.

The electrophilic activator is preferably present during the reaction in an amount of 100 to 150 mol%, more preferably 100 to 110 mol%, based on the molar amount of the carbonyl compound A2 as 100 mol%.

The soft nucleophile as an additive, if present during the reaction, is preferably used in an amount of 100 to 200 mol%, more preferably of 100 to 120 mol%, based on the molar amount of the sulfoxide compound S1 as 100 mol%.

The reaction can be conveniently accomplished by contacting the carbonyl compound A2 (and its preferred variants discussed above) and the electrophilic activator in a suitable solvent, preferably an aprotic solvent, such as CH₂Cl₂, for a sufficient extent, typically 5 to 30 minutes, before the sulfoxide compound S1 (and its preferred variants discussed above), and optionally the soft nucleophile are added to the mixture. The reaction temperature can be suitable adjusted, typically the reaction is carried out at a temperature in the range of -10 °C to 50 °C, preferably in the range of 0 °C to 25 °C. Once the reaction has been allowed to proceed to a sufficient extent, e.g. over 1 to 10 hours, the obtained product can be isolated and, if necessary, purified by conventional methods.

In accordance with a third embodiment of the method in accordance with the invention, an alpha-substituted carbonyl compound or an alpha-substituted nitrile compound carrying an aryl or heteroaryl group as an alpha-substituent is provided by reacting the sulfoxide compound S1 with a nucleophile A3, which is selected from a silyl enolether A3a, an α-silyl nitrile A3b, an α-stannyl nitrile A3c, and a β-ketoester derivative A3d, in the presence of an electrophilic activator.

A preferred structure of the silyl enolether can be illustrated by the following formula: 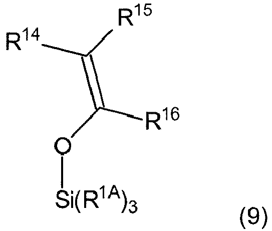 wherein
R^{1A}, independently for each occurrence, is a hydrocarbyl group, preferably an alkyl group; and R¹⁴ to R¹⁶ are independently selected from hydrogen and an organyl group.

The reaction of the preferred sulfoxide of formula (1) with the preferred silyl enolether of formula (9) to yield an alpha-substituted carbonyl compound in accordance with the method of the present invention can thus be illustrated by the following reaction scheme: 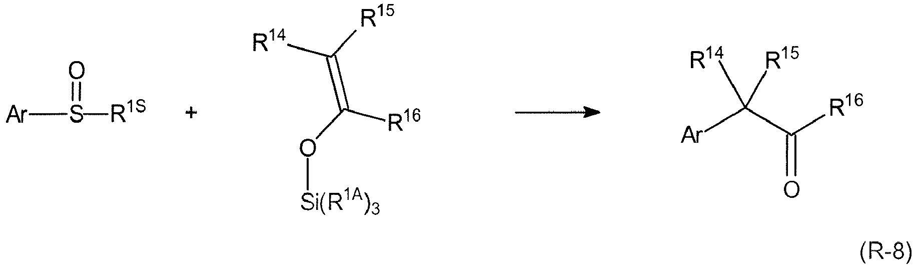 wherein the variables Ar, R^{1S}, R¹⁴ to R¹⁶ and R^{1A} are as defined above.

A preferred structure of the α-silyl nitrile and the α-stannyl nitrile can be illustrated by the following formula: 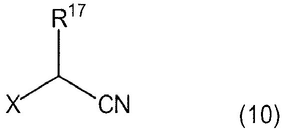 wherein
X is selected from -Si(R^{2A})₃ and -Sn(R^{3A})₃; R^{2A} and R^{3A} are, independently for each occurrence, a hydrocarbyl group, preferably an alkyl group; and R¹⁷ is selected from hydrogen and an organyl group.

The reaction of the preferred sulfoxide of formula (1) with the preferred α-silyl nitrile or the α-stannyl nitrile of formula (10) to yield an alpha-substituted nitrile compound in accordance with the method of the present invention can thus be illustrated by the following reaction scheme: 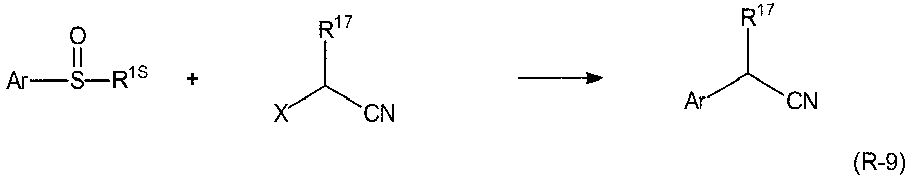 wherein the variables Ar, R^{1S}, X and R¹⁷ are as defined above.

A preferred structure of the β-ketoester derivative can be illustrated by the following formula: 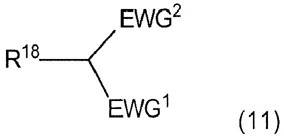 wherein
EWG¹ and EWG² are the same or different, preferably the same, and are selected from -C(O)R^{4A}, -C(O)OR^{5A}, -C(O)NR^{6A}R^{7A}, and -CN; and R¹⁸ is selected from hydrogen and an organyl group. R^{4A}, R^{5A}, R^{6A} and R^{7A} are independently selected from hydrogen and a hydrocarbyl group, preferably from hydrogen and an alkyl group.

The reaction of the preferred sulfoxide of formula (1) with the preferred β-ketoester derivative of formula (11) to yield an alpha-substituted carbonyl compound or an alpha-substituted nitrile compound in accordance with the method of the present invention can thus be illustrated by the following reaction scheme: 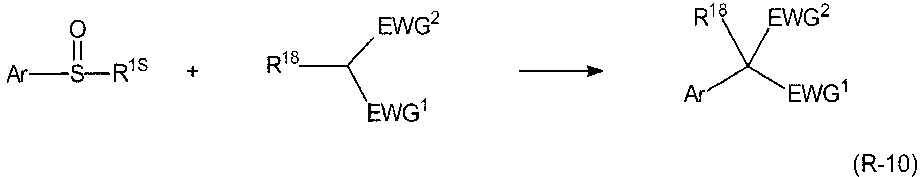 wherein the variables Ar, R^{1S}, R¹⁸, EWG¹ and EWG² are as defined above.

The reaction of the sulfoxide compound S1 (and its preferred variants discussed above) with the nucleophile A3 (and its preferred variants discussed above) is carried out in the presence of an electrophilic activator.

Examples of a suitable electrophilic activators are trifluoromethanesulfonic acid anhydride (triflic anhydride, Tf₂O), trifluoroacetic acid anhydride (TFAA), toluenesulfonic anhydride (Ts₂O), methanesulfonic anhydride (Ms₂O), and TfOC(O)CF₃.

Preferably, the reaction of the sulfoxide compound S1 (and its preferred variants discussed above) with the nucleophile A3 (and its preferred variants discussed above) is carried out in the further presence of a soft nucleophile as an additive which assists in obtaining the product at a high yield and in reducing the byproduct formation. As exemplary classes of compounds which can be used as soft nucleophiles, reference can be made to thiol compounds, such as alkylthiols or arylthiols, and to alkenes, arenes and heteroarenes with a N-Parameter higher than 3.5 according to the Mayr's Database of Reactivity Parameters (e.g. https://www.cup.Imu.de/oc/mayr/reaktionsdatenbank). Exemplary compounds which can be suitably used as such an additive (a soft nucleophile) include octanethiol, phenylthiol, 2-methyl-2-butene, and 2,3-dimethylbut-2-ene.

For the reaction, the sulfoxide compound S1 (and its preferred variants discussed above) and the nucleophile A3 (and its preferred variants discussed above) are typically used in a molar ratio ranging from 2/1 to 1/2, and preferably at a molar ratio of about 1/1.0-1.5.

The electrophilic activator is preferably present during the reaction in an amount of 100 to 150 mol%, more preferably 100 to 110 mol%, based on the molar amount of the sulfoxide compound S1 as 100 mol%.

The soft nucleophile as an additive, if present during the reaction, is preferably used in an amount of 100 to 500 mol%, more preferably of 100 to 150 mol%, based on the molar amount of the sulfoxide compound S1 as 100 mol%.

The reaction can be conveniently accomplished by contacting the sulfoxide compound S1 (and its preferred variants discussed above), the nucleophile A3 (and its preferred variants discussed above), the electrophilic activator and optionally the soft nucleophile in a suitable solvent, preferably an aprotic solvent, such as CH₂Cl₂, nitromethane or toluene. The reaction temperature can be suitable adjusted, typically the reaction is carried out at a temperature in the range of -10 °C to 50 °C, preferably in the range of 0 °C to 25 °C. Once the reaction has been allowed to proceed to a sufficient extent, e.g. over 1 to 10 hours, the obtained product can be isolated and, if necessary, purified by conventional methods.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

### Examples

### General Information

*General procedures:* All reactions were performed in round bottom flasks or vials fitted with rubber septa with magnetic stirring under an atmosphere of argon unless otherwise stated. Liquids and solutions were transferred via syringe. All reactions were performed using anhydrous solvents from Acros Organics or Sigma-Aldrich. Reaction progress was monitored by thin layer chromatography (TLC) performed on aluminum plates coated with silica gel F₂₅₄ with 0.2 mm thickness. Chromatograms were visualized by fluorescence quenching with UV light at 254 nm or by staining using potassium permanganate followed by heating. Flash column chromatography was carried out on 230-400 mesh silica gel (Merck and Co.) using reagent grade solvents.

*Materials:* All commercial reagents and solvents were used without further purification. Racemic products were synthesized according to the same procedure from the racemic sulfoxides.

*Instrumentation:* ¹H-NMR and ¹³C-NMR spectra were recorded using Bruker AV-400 or AV-600 spectrometers at 300 K. Chemical shifts (*δ*) were given in parts per million (ppm), referenced to the solvent peak of CDCl₃, defined at *δ* = 7.26 ppm (¹H-NMR) and *δ* = 77.16 ppm (¹³C-NMR). Coupling constants (*J*) are reported in Hertz (Hz). ¹H-NMR splitting patterns were designated as singlet (s), doublet (d), triplet (t), quartet (q) and septet (sept) as they appeared in the spectrum. If the appearance of a signal differed from the expected splitting pattern, the observed pattern was designated as apparent (app). Splitting patterns that could not be interpreted or easily visualized were designated as multiplet (m) or broad (br). Mass spectra were obtained using a Finnigan MAT 8200 or (70 eV) or an Agilent 5973 (70 eV) spectrometer, using electrospray ionization (ESI). Infrared (IR) spectra were obtained using Perkin-Elmer Spectrum 100 FT-IR spectrometer. Wavenumbers (*v* = *λ*⁻¹) are reported in cm⁻¹.

### Synthesis of Ynamides

Following the method developed by Stahl (J. Am. Chem. Soc. 2008, 130, 833-835), to a 100 mL flask were added CuCl₂ (20 mol%), 2-oxazolidone (5.0 equiv) and Na₂CO₃ (2.0 equiv). The reaction flask was purged with oxygen (balloon) for 15 min, after which pyridine (2.0 equiv) and dry toluene (5 mL mmol⁻¹) was added. A balloon filled with oxygen was connected to the flask and the reaction mixture was heated at 70-80 °C with stirring. After 15 min, a solution of the alkyne (1.0 equiv) in dry toluene (5 mL mmol⁻¹) was added over 2 h using dropping funnel. After this addition, the mixture was stirred at 80 °C for another 12-24 h and was then cooled to rt. The reaction was interrupted by addition of NH₄Cl solution (saturated in H₂O) and diluted with diethyl ether. The organic layer was separated and dried with Na₂SO₄. The dried solution was filtered and concentrated under reduced pressure. The residue was purified by flash chromatography on silica gel with hexane/ethyl acetate.

Further information on the preparation and characterization of ynamides is provided in Angew. Chem. Int. Ed. 2014, 53, 8718-8721; Angew. Chem. Int. Ed. 2017, 56, 2212-2215; Science 2018, 361, 664-667.

### Synthesis of Sulfoxides

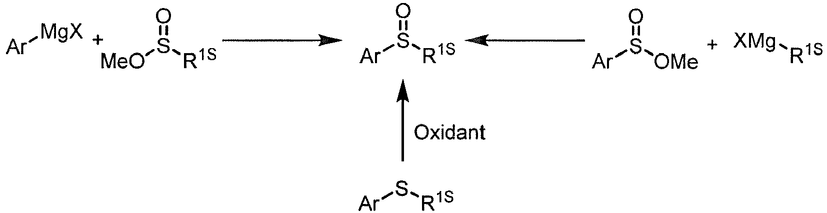

Sulfoxides are commonly obtained by oxidation using an oxidant such as mCPBA or NaIO₄ from the respective sulfides or by organometallic addition to the respective sulfinate species. Further information on the preparation and characterization of sulfoxides is provided in Angew. Chem. Int. Ed. 2017, 56, 2212-2215; Chem. Sci. 2018, 9, 4124-4131.

### α-Arylation

*General procedure:* A solution of sulfoxide (0.20 mmol, 1.0 equiv), ynamide (0.24 mmol, 1.2 equiv) and n-octanethiol (21 µL, 0.20 mmol, 1.0 equiv) in CH₂Cl₂ (2 mL, 10 mL mmol⁻¹) was cooled to 0 °C. To the cooled solution, a second solution of Tf₂NH in CH₂Cl₂ (0.2 mL of a 0.1 M solution, 0.02 mmol, 10 mol%) was added via syringe and the reaction was stirred at 0 °C until TLC analysis showed that the starting material had been consumed (usually 3 hours). Solid NaHCO₃ (20 mg) was then added and the reaction mixture was stirred at rt for 5 minutes. The suspension was filtered over a plug of cotton and the solvent evaporated. The crude residue was purified by column chromatography (heptane:ethyl acetate 3:1) to afford the arylated product.

### 3-(2-(2,6-Dimethoxyphenyl)hexanoyl)oxazolidin-2-one

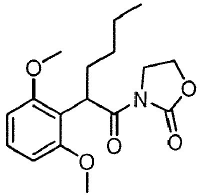

Performed on a 0.2 mmol scale delivering the product in 85% yield as white crystals. ¹H-NMR (400 MHz, CDCl₃): *δ* 7.13 (t, *J* = 8.3 Hz, 1H), 6.51 (d, *J* = 8.3 Hz, 2H), 4.75 (t, *J* = 6.8 Hz, 1H), 4.28 (app td, *J* = 8.9, 4.8 Hz, 1H), 4.15 (app q, *J* = 8.7 Hz, 1H), 4.04 (app dd, *J* = 19.4, 9.2 Hz, 1H), 3.83 (ddd, *J* = 10.6, 8.8, 4.8 Hz, 1H), 3.78 (s, 6H), 2.16-2.05 (m, 1H), 1.67-1.51 (m, 1H), 1.43-1.14 (m, 4H), 0.86 (t, *J* = 7.1 Hz, 3H); **¹³C-NMR** (101 MHz, CDCl₃): *δ* 175.0, 157.9 (2C), 152.6, 127.8, 117.7, 104.4 (2C), 61.8, 55.89, 55.87, 43.2, 41.4, 30.3, 29.5, 22.7, 13.9; **HRMS** (ESI+): exact mass calculated for [M+H]⁺ (C₁₇H₂₃NO₅Na⁺) requires *m*/*z* 344.1468, found *m*/*z* 344.1467; **IR** (thin film): *v* 2949, 2930, 1772, 1701, 1591, 1471, 1228, 1105, 1037, 724 cm⁻¹.

### 3-(2-(2,6-Dimethoxyphenyl)-5-phenylpentanoyl)oxazolidin-2-one

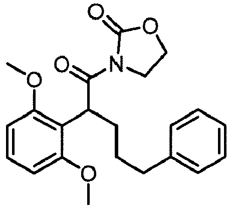

Performed on a 0.2 mmol scale delivering the product in 94% yield as white crystals.
**¹H-NMR** (400 MHz, CDCl₃): *δ* 7.30-7.21 (m, 2H), 7.20-7.11 (m, 4H), 6.53 (d, *J* = 8.3 Hz, 2H), 4.83 (t, *J* = 6.5 Hz, 1H), 4.33-4.22 (m, 1H), 4.16 (app q, *J* = 8.7 Hz, 1H), 4.05 (app dt, *J* = 18.2, 9.1 Hz, 1H), 3.84 (ddd, *J* = 10.6, 8.8, 4.8 Hz, 1H), 3.78 (s, 6H), 2.75-2.54 (m, 2H), 2.26-2.10 (m, 1H), 1.85-1.53 (m, 3H); **¹³C-NMR** (101 MHz, CDCl₃): *δ* 174.8, 157.9 (2C), 152.5, 142.7, 128.3 (2C), 128.0 (2C), 127.9, 125.4, 117.3, 104.4 (2C), 61.8, 55.9 (2C), 43.2, 41.3, 35.9, 30.2, 28.7; **HRMS** (ESI+): exact mass calculated for [M+H]⁺ (C₂₂H₂₆NO₅⁺) requires *m*/*z* 406.1625, found *m*/*z* 406.1626; **IR** (thin film): *v* 2933, 1777, 1702, 1592, 1473, 1241, 1103, 1038, 728, 701 cm⁻¹.

### 2-(2,6-Dimethoxyphenyl)-N-methyl-5-phenyl-N-tosylpentanamide

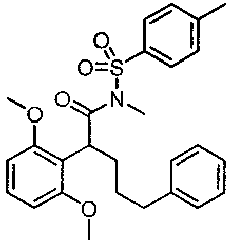

Performed on a 0.2 mmol scale delivering the product in 70% yield as colorless oil. **¹H-NMR** (400 MHz, CDCl₃): *δ* 7.85 (d, *J* = 8.3 Hz, 2H), 7.29 (d, *J* = 8.0 Hz, 2H), 7.24-7.17 (m, 3H), 7.15-7.04 (m, 3H), 6.51 (d, *J* = 8.4 Hz, 2H), 4.11 (dd, *J* = 8.4, 5.4 Hz, 1H), 3.64 (s, 6H), 2.98 (s, 3H), 2.62-2.44 (m, 2H), 2.42 (s, 3H), 2.14-2.03 (m, 1H), 1.75-1.63 (m, 1H), 1.61-1.48 (m, 1H), 1.46-1.33 (m, 1H); **¹³C-NMR** (101 MHz, CDCl₃): *δ* 174.5, 157.7 (2C), 143.8, 142.6, 136.8, 129.0 (2C), 128.7, 128.6 (2C), 128.3 (2C), 128.1 (2C), 125.4, 114.9, 103.8 (2C), 55.51, 55.50, 41.5, 35.8, 32.6, 29.9, 28.4, 21.5; **HRMS** (ESI+): exact mass calculated for [M+H]⁺ (C₂₇H₃₂NO₅S⁺) requires *m*/*z* 482.1996, found *mlz* 482.1994; **IR** (thin film): *v* 2934, 1703, 1593, 1474, 1349, 1241, 1164, 1104, 909, 727 cm⁻¹.

### 3-(2-(2,6-Dimethoxyphenyl)-4-methylpentanoyl)oxazolidin-2-one

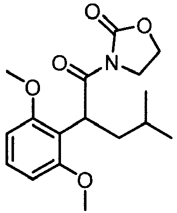

Performed on a 0.2 mmol scale delivering the product in 72% yield as colorless foam.
**¹H-NMR** (400 MHz, CDCl₃): *δ* 7.13 (t, *J* = 8.3 Hz, 1H), 6.51 (d, *J* = 8.3 Hz, 2H), 4.87 (dd, *J* = 7.4, 6.4 Hz, 1H), 4.29 (td, *J* = 9.0, 5.0 Hz, 1H), 4.17 (app q, *J* = 8.7 Hz, 1H), 4.09-4.01 (m, 1H), 3.85 (ddd, *J* = 10.6, 8.9, 5.0 Hz, 1H), 3.78 (s, 6H), 2.10 (ddd, *J* = 13.8, 7.4, 6.7 Hz 1H), 1.58 (app sept, 6.6 Hz, 1H), 1.39 (app dt, *J* = 13.3, 6.6 Hz, 1H), 0.94 (d, *J* = 6.5 Hz, 3H), 0.90 (d, *J* = 6.5 Hz, 3H); **¹³C-NMR** (176 MHz, CDCl₃): *δ* 175.2, 158.0 (2C), 152.7, 128.0, 118.1, 104.5 (2C), 61.9, 56.0 (2C), 43.4, 40.0, 39.9, 26.0, 23.0, 22.7; **HRMS** (ESI+): exact mass calculated for [M+Na]⁺ (C₁₇H₂₃NNaO₅⁺) requires *m*/*z* 344.1468, found *m*/*z* 344.1467; **IR** (thin film): *v* 2954, 1775, 1701, 1592, 1473, 1235, 1105, 1088, 726 cm⁻¹.

### 3-(2-Cyclohexyl-2-(2,6-dimethoxyphenyl)acetyl)oxazolidin-2-one

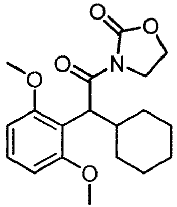

Performed on a 0.2 mmol scale delivering the product in 79% yield as colorless oil.
**¹H-NMR** (600 MHz, CDCl₃): *δ* 7.14 (t, *J* = 8.3 Hz, 1H), 6.50 (d, *J* = 8.3 Hz, 2H), 4.68 (d, *J* = 7.5 Hz, 1H), 4.26 (ddd, *J* = 8.9, 8.4, 4.3 Hz, 1H), 4.12 (dd, *J* = 17.4, 8.5 Hz, 1H), 4.08-3.97 (m, 1H), 3.84-3.75 (m, 7H), 2.30-2.17 (m, 1H), 1.94-1.85 (m, 1H), 1.74-1.56 (m, 3H), 1.39-1.03 (m, 5H), 0.96 (app qd, *J* = 12.5, 3.1 Hz, 1H); **¹³C-NMR** (151 MHz, CDCl₃): *δ* 174.7, 158.8 (2C), 152.7, 128.2, 115.7, 104.3 (2C), 61.9, 56.0 (2C), 46.9, 43.5, 38.6, 32.7, 29.6, 26.0, 26.9, 26.7; **HRMS** (ESI+): exact mass calculated for [M+Na]⁺ (C₁₉H₂₅NNaO₅⁺) requires *m*/*z* 370.1625, found *m*/*z* 370.1622; **IR** (thin film): *v* 2925, 2850, 1777, 1702, 1592, 1474, 1239, 1104, 731 cm⁻¹.

### 3-(2-(2,4,6-Trimethoxyphenyl)hexanoyl)oxazolidin-2-one

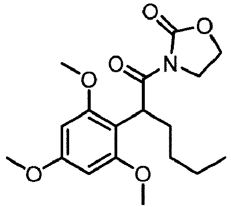

Performed on a 0.2 mmol scale delivering the product in 72% yield as colorless oil.
**¹H-NMR** (700 MHz, CDCl₃): *δ* 6.09 (s, 2H), 4.67 (t, *J* = 6.9 Hz, 1H), 4.28 (td, *J* = 9.0, 4.6 Hz, 1H), 4.17 (app q, *J* = 8.7 Hz, 1H), 4.04 (dt, *J* = 10.6, 9.3 Hz, 1H), 3.82 (ddd, *J* = 10.6, 8.9, 4.7 Hz, 1H), 3.77 (s, 3H), 3.76 (s, 6H), 2.10-2.00 (m, 1H), 1.63-1.55 (m, 1H), 1.36-1.16 (m, 4H), 0.85 (t, *J* = 7.1 Hz, 3H); **¹³C-NMR** (176 MHz, CDCl₃): *δ* 175.5, 160.1, 158.8 (2C), 152.8, 110.3 (2C), 91.2, 62.0, 56.0 (2C), 55.3, 43.4, 41.3, 30.6, 29.6, 22.9, 14.1; **HRMS** (ESI+): exact mass calculated for [M+Na]⁺ (C₁₈H₂₅NNaO₆⁺) requires *m*/*z* 374.1574, found *m*/*z* 374.1573; **IR** (thin film): *v* 2994, 1777, 1702, 1606, 1592, 1203, 1119, 816 cm⁻¹.

### 5-(2,6-Dimethoxyphenyl)-6-oxo-6-(2-oxooxazolidin-3-yl)hexanenitrile

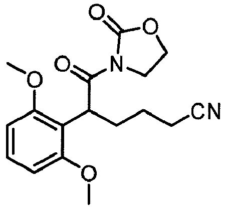

Performed on a 0.2 mmol scale delivering the product in 75% yield as colorless oil.
**¹H-NMR** (700 MHz, CDCl₃): *δ* 7.17 (t, *J* = 8.3 Hz, 1H), 6.53 (d, *J* = 8.3 Hz, 2H), 4.79 (t, *J* = 6.7 Hz, 1H), 4.33 (td, *J* = 9.0, 4.5 Hz, 1H), 4.20 (app q, *J* = 8.9 Hz, 1H), 4.07 (dt, *J* = 10.6, 9.3 Hz, 1H), 3.85 (ddd, *J* = 10.6, 9.0, 4.6 Hz, 1H), 3.80 (s, 6H), 2.39-2.28 (m, 2H), 2.21-2.14 (m, 1H), 1.87-1.80 (m, 1H), 1.75-1.60 (m, 2H); **¹³C-NMR** (176 MHz, CDCl₃): *δ* 174.7, 158.0 (2C), 152.8, 128.7, 120.0, 116.2, 104.5 (2C), 62.1, 56.1 (2C), 43.4, 40.7, 29.6, 23.2, 17.3; HRMS (ESI+): exact mass calculated for [M+Na]⁺ (C₁₇H₂₀N₂NaO₅⁺) requires *m*/*z* 355.1264, found *mlz* 355.1254; **IR** (thin film): *v* 2928, 2245, 1776, 1700, 1593, 1474, 1104, 726 cm⁻¹.

### 3-(2-(2,6-Dimethoxyphenyl)-3-hydroxypropanoyl)oxazolidin-2-one

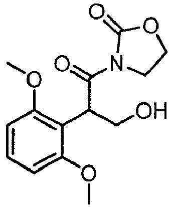

Performed on a 0.2 mmol scale using 3-(3-((tert-butyldimethylsilyl)oxy)prop-1-yn-1-yl)oxazolidin-2-one. The acid catalyst was quenched by the addition of a saturated aqueous solution of NaHCO₃ (1 mL) and was stirred for 30 min at rt before being extracted with dichloromethane (3 × 2 mL). The organic phases were combined, dried over MgSO₄, filtered and concentrated in vacuo. Purification using flash chromatography on silica gel with hexane/ethyl acetate afforded the product in 85% yield as colorless solid.
**¹H-NMR** (700 MHz, CDCl₃): *δ* 7.17 (t, *J* = 8.3 Hz, 1H), 6.53 (d, *J* = 8.3 Hz, 2H), 4.95 (dd, *J* = 7.8, 3.9 Hz, 1H), 4.34 (td, *J* = 9.3, 4.9 Hz, 1H), 4.24 (app q, *J* = 8.8 Hz, 1H), 4.10-4.02 (m, 2H), 3.93-3.88 (m, 1H), 3.79 (s, 6H), 3.76-3.72 (m, 1H), 2.93 (dd, *J* = 8.5, 5.7 Hz, 1H); **¹³C-NMR** (176 MHz, CDCl₃): *δ* 175.6, 158.1 (2C), 152.5, 128.9, 113.5, 104.6 (2C), 62.4, 62.2, 56.2 (2C), 44.8, 42.9; **HRMS** (ESI+): exact mass calculated for [M+Na]⁺ (C₁₄H₁₇NNaO₆⁺) requires *m*/*z* 318.0948, found *m*/*z* 318.0942; **IR** (thin film): *v* 3534, 2939, 1778, 1692, 1594, 1475, 1105, 1037, 734 cm⁻¹.

### 2-(4-(2,6-Dimethoxyphenyl)-5-oxo-5-(2-oxooxazolidin-3-yl)pentyl)isoindoline-1,3-dione

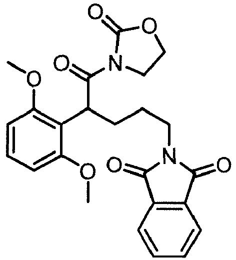

Performed on a 0.2 mmol scale delivering the product in 75% yield as colorless foam.
**¹H-NMR** (700 MHz, CDCl₃): *δ* 7.81 (dd, *J* = 5.4, 3.0 Hz, 2H), 7.68 (dd, *J* = 5.4, 3.0 Hz, 2H), 7.13 (t, *J* = 8.3 Hz, 1H), 6.49 (d, *J* = 8.3 Hz, 2H), 4.79 (t, *J* = 6.5 Hz, 1H), 4.30 (td, *J* =9.2, 4.8 Hz, 1H), 4.18 (app q, *J* = 8.9 Hz, 1H), 4.07-4.01 (m, 1H), 3.86-3.81 (m, 1H), 3.76 (s, 6H), 3.68 (t, *J* = 7.0 Hz, 2H), 2.16-2.10 (m, 1H), 1.80-1.72 (m, 2H), 1.69-1.62 (m, 1H); ¹³C-NMR (176 MHz, CDCl₃): *δ* 174.8, 168.6 (2C), 158.1 (2C), 152.8, 133.9 (2C), 132.4 (2C), 128.4, 123.3 (2C), 116.7, 104.5 (2C), 62.0, 56.0 (2C), 43.4, 41.2, 38.4, 27.8, 26.2; HRMS (ESI+): exact mass calculated for [M+Na]⁺ (C₂₄H₂₄N₂NaO₇⁺) requires *m*/*z* 475.1476, found *mlz* 475.1465; **IR** (thin film): *v* 2928, 1775, 1706, 1593, 1105, 721 cm⁻¹.

### 3-(2-(2,4-Dichloro-6-methoxyphenyl)hexanoyl)oxazolidin-2-one

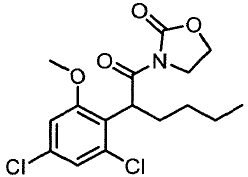

Performed on a 0.1 mmol scale delivering the product in 75% yield as colorless viscous liquid.
**¹H-NMR** (600 MHz, CDCl₃): *δ* 7.02 (d, *J* = 2.0 Hz, 1H), 6.75 (d, *J* = 2.0 Hz, 1H), 4.78 (dd, *J* = 7.6, 6.0 Hz, 1H), 4.36 (td, *J* = 9.1, 4.9 Hz, 1H), 4.26 (app q, *J* = 8.8 Hz, 1H), 4.11 (dt, *J* = 10.9, 9.1 Hz, 1H), 3.89 (ddd, *J* = 10.7, 8.9, 4.9 Hz, 1H), 3.78 (s, 3H), 2.17 (dddd, *J* = 13.0, 10.3, 7.6, 4.9 Hz, 1H), 1.64 (ddt, *J* = 13.5, 11.0, 5.6 Hz, 1H), 1.41 (dddd, *J* = 16.5, 13.9, 7.8, 4.1 Hz, 1H), 1.36-1.29 (m, 2H), 1.29-1.19 (m, 1H), 0.88 (t, *J* = 7.2 Hz, 3H); **¹³C-NMR** (151 MHz, CDCl₃): *δ* 173.3, 158.4, 152.8, 136.0, 133.4, 126.4, 122.4, 110.8, 62.2, 56.4, 45.1, 43.4, 30.2, 29.7, 22.9, 14.1; **HRMS** (ESI+): exact mass calculated for [M+Na]⁺ (C₁₆H₁₉Cl₂NNaO₄⁺) requires *m*/*z* 382.0583, found *m*/*z* 382.0587; **IR** (thin film): *v* 2956, 2930, 2870, 2860, 1782, 1707, 1582, 1567, 1042 cm⁻¹.

### 3-(2-(2,4-Dichloro-6-methoxyphenyl)propanoyl)oxazolidin-2-one

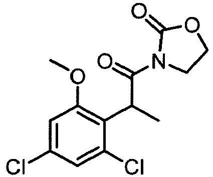

Performed on a 0.1 mmol scale delivering the product in 87% yield as colorless foam.
**¹H-NMR** (600 MHz, CDCl₃):*δ* 7.01 (d, *J* = 2.0 Hz, 1H), 6.74 (d, *J* = 2.0 Hz, 1H), 4.80 (q, *J* = 7.0 Hz, 1H), 4.40-4.33 (m, 1H), 4.29-4.22 (m, 1H), 4.11 (dt, *J* = 10.9, 9.2 Hz, 1H), 3.92-3.85 (m, 1H), 3.78 (s, 3H), 1.39 (d, *J* = 7.0 Hz, 3H); **¹³C-NMR** (151 MHz, CDCl₃):*δ* 173.9, 157.8, 152.9, 135.5, 133.3, 127.5, 122.4, 110.9, 62.3, 56.5, 43.3, 40.2, 15.1; **HRMS** (ESI+): exact mass calculated for [M+Na]⁺ (C₁₃H₁₃Cl₂NNaO₄⁺) requires *m*/*z* 340.0114, found *m*/*z* 340.0113; **IR** (thin film): *v* 3089, 2984, 2937, 2870, 2855, 1777, 1704, 1582, 1567, 1039 cm⁻¹.

### 3-(2-(2-Bromo-4,6-dimethoxyphenyl)hexanoyl)oxazolidin-2-one

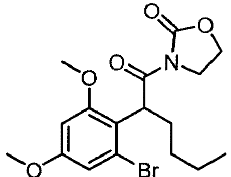

Performed on a 0.12 mmol scale delivering the product in 84% yield as a colorless crystals.
**¹H-NMR** (400 MHz, CDCl₃): *δ* 6.74 (d, *J* = 2.5 Hz, 1H), 6.36 (d, *J* = 2.5 Hz, 1H), 4.71 (dd, *J* = 7.9, 5.5 Hz, 1H), 4.33 (td, *J* = 9.3, 4.9 Hz, 1H), 4.22 (app q, *J* = 8.7 Hz, 1H), 4.13-4.02 (m, 1H), 3.92-3.83 (m, 1H), 3.76 (s, 3H), 3.72 (s, 3H), 2.21-2.09 (m, 1H), 1.71-1.60 (m, 1H), 1.49-1.38 (m, 1H), 1.37-1.19 (m, 3H), 0.88 (t, *J* = 7.1 Hz, 3H); **¹³C-NMR** (101 MHz, CDCl₃): *δ* 173.7, 159.2, 158.3, 152.6, 126.6, 121.6, 109.9, 98.8, 62.0, 55.9, 55.7, 47.5, 43.3, 30.5, 29.7, 22.8, 14.0; **HRMS** (ESI+): exact mass calculated for [M+Na]⁺ (C₁₇H₂₂NO₅BrNa⁺) requires *m*/*z* 422.0574, found *m*/*z* 422.0573; **IR** (thin film): *v* 2956, 1780, 1705, 1602, 1566, 1385, 1213, 1032 cm⁻¹.

### 3-(2-(2-Chloro-4,6-dimethoxyphenyl)hexanoyl)oxazolidin-2-one

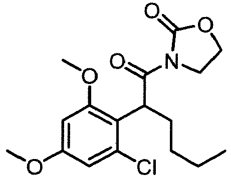

Performed on a 0.12 mmol scale delivering the product in 72% yield as a colorless oil.
**¹H-NMR** (700 MHz, CDCl₃): *δ* 6.54 (d, *J* = 2.4 Hz, 1H), 6.32 (d, *J* = 2.4 Hz, 1H), 4.76-4.72 (m, 1H), 4.34 (td, *J* = 9.1, 4.7 Hz, 1H), 4.23 (app q, *J* = 8.8 Hz, 1H), 4.09 (app dt, *J* = 18.6, 9.2 Hz, 1H), 3.88 (ddd, *J* = 10.7, 9.0, 4.8 Hz, 1H), 3.77 (s, 3H), 3.74 (s, 3H), 2.19-2.11 (m, 1H), 1.68-1.62 (m, 1H), 1.44-1.37 (m, 1H), 1.37-1.29 (m, 2H), 1.29-1.21 (m, 1H), 0.87 (t, *J* = 7.3 Hz, 3H); **¹³C-NMR** (176 MHz, CDCl₃): *δ* 174.0, 159.1, 158.6, 152.6, 135.8, 119.9, 106.5, 98.1, 62.0, 55.9, 55.4, 44.8, 43.3, 30.4, 29.5, 22.8, 14.0; **HRMS** (ESI+): exact mass calculated for [M+Na]⁺ (Cₗ₇H₂₂NO₅ClNa⁺) requires *mlz* 378.1079, found *m*/*z* 378.1078; **IR** (thin film): *v* 2956, 1780, 1705, 1604, 1573, 1385, 1213, 1041 cm⁻¹.

### 3-(2-(2-(Chloromethyl)-4,6-dimethoxyphenyl)hexanoyl)oxazolidin-2-one

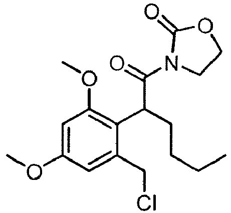

Performed on a 0.1 mmol scale delivering the product in 57% yield as a pale-yellow oil.
**¹H-NMR** (600 MHz, CDCl₃): *δ* 6.56 (d, *J* = 2.5 Hz, 1H), 6.39 (d, *J* = 2.5 Hz, 1H), 4.91 (d, *J* = 11.9 Hz, 1H), 4.63-4.53 (m, 2H), 4.33 (td, *J* = 9.1, 4.5 Hz, 1H), 4.20 (app q, *J* = 8.9 Hz, 1H), 4.08 (app dt, *J* = 18.7, 9.3 Hz, 1H), 3.92-3.85 (m, 1H), 3.8₀ (s, 3H), 3.74 (s, 3H), 2.26-2.18 (m, 1H), 1.69-1.62 (m, 1H), 1.49-1.40 (m, 1H), 1.38-1.22 (m, 3H), 0.87 (t, *J* = 7.2 Hz, 3H); **¹³C-NMR** (151 MHz, CDCl₃): *δ* 174.4, 159.2, 158.4, 152.9, 138.3, 120.4, 107.0, 99.4, 62.0, 55.7, 55.3, 44.8, 44.0, 43.4, 31.3, 30.2, 22.8, 13.9; **HRMS** (ESI+): exact mass calculated for [M+Na]⁺ (C₁₈H₂₄NO₅ClNa⁺) requires *mlz* 392.1235, found *m*/*z* 392.1243; **IR** (thin film): *v* 2957, 1777, 1707, 1606, 1463, 1385, 1222, 1202, 1155, 760 cm⁻¹.

### 3-(2-(2-Methoxynaphthalen-1-yl)hexanoyl)oxazolidin-2-one

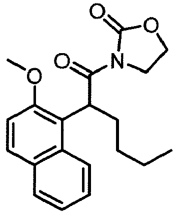

Performed on a 0.11 mmol delivering the product in 75% yield as a colorless oil.
**¹H-NMR** (400 MHz, CDCl₃): *δ* 8.04 (d, *J* = 8.6 Hz, 1H), 7.80-7.71 (m, 2H), 7.49 (ddd, *J* = 8.5, 6.8, 1.4 Hz, 1H), 7.34 (ddd, *J* = 8.0, 6.8, 1.0 Hz, 1H), 7.21 (d, *J* = 9.0 Hz, 1H), 5.21 (dd, *J* = 8.0, 5.6 Hz, 1H), 4.34-4.21 (m, 1H), 4.18-4.05 (m, 2H), 3.96-3.82 (m, 4H), 2.40-2.25 (m, 1H), 1.82-1.69 (m, 1H), 1.53-1.42 (m, 1H), 1.39-1.25 (m, 3H), 0.86 (t, *J* = 7.1 Hz, 3H); **¹³C-NMR** (101 MHz, CDCl₃): *δ* 175.1, 154.3, 152.7, 133.0, 129.7, 129.1, 128.6, 126.6, 123.5, 123.3, 122.4, 114.2, 61.8, 57.0, 43.6, 43.2, 31.3, 30.1, 22.9, 13.9; **HRMS** (ESI+): exact mass calculated for [M+Na]⁺ (C₂₀H₂₃NO₄Na⁺) requires *m*/*z* 364.1519, found *m*/*z* 364.1518; IR (thin film): *v* 2956, 1779, 1704, 1385, 1259, 750 cm⁻¹.

### 3-(2-(4-Methoxy-2,6-dimethylphenyl)hexanoyl)oxazolidin-2-one

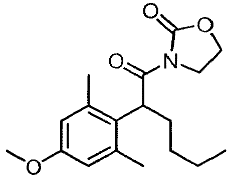

Performed on a 0.11 mmol scale delivering the product in 73% yield as a pale-yellow oil.
**¹H-NMR** (400 MHz, CDCl₃): *δ* 6.52 (s, 2H), 4.68 (dd, *J* = 9.0, 4.5 Hz, 1H), 4.40-4.32 (m, 1H), 4.25 (app q, *J* = 8.8 Hz, 1H), 4.10 (dt, *J* = 10.8, 9.1 Hz, 1H), 3.94 (ddd, *J* = 10.9, 8.9, 4.7 Hz, 1H), 3.74 (s, 3H), 2.39-2.23 (m, 7H), 1.60-1.42 (m, 2H), 1.39-1.29 (m, 2H), 1.29-1.15 (m, 1H), 0.89 (t, *J* = 7.2 Hz, 3H); **¹³C-NMR** (101 MHz, CDCl₃): *δ* 175.3, 157.5, 152.5, 138.5 (br, 2C), 129.3, 114.4 (2C), 61.8, 54.9, 46.5, 43.4, 30.9, 30.5, 22.8, 21.1 (2C), 14.0; **HRMS** (ESI+): exact mass calculated for [M+Na]⁺ (C₁₈H₂₅NO₄Na⁺) requires *m*/*z* 342.1676, found *m*/*z* 342.1676; **IR** (thin film): *v* 2958, 1783, 1697, 1603, 1385, 1195 cm⁻¹.

### 3-(2-(2, 4-Di-tert-butyl-6-methoxyphenyl)hexanoyl)oxazolidin-2-one

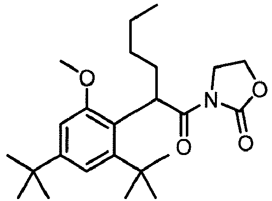

Performed on a 0.09 mmol scale delivering the product in 49% yield as white crystals.
**¹H-NMR** (400 MHz, CDCl₃): *δ* 7.08 (d, *J* = 1.9 Hz, 1H), 6.85 (d, *J* = 1.9 Hz, 1H), 5.49 (dd, *J* = 10.0, 5.3 Hz, 1H), 4.40 (dd, *J* = 8.7, 7.4 Hz, 2H), 4.10-3.94 (m, 2H), 3.80 (s, 3H), 2.48-2.36 (m, 1H), 2.04 (tt, *J* = 12.3, 4.9 Hz, 1H), 1.39 (s, 9H), 1.32 (s, 9H), 1.42-1.14 (m, 3H), 1.07-0.95 (m, 1H), 0.82 (t, *J* = 7.3 Hz, 3H); **¹³C-NMR** (101 MHz, CDCl₃): *δ* 173.9, 160.4, 153.3, 149.9, 149.3, 121.7, 116.6, 108.2, 61.9, 56.0, 45.2, 43.4, 36.2, 34.9, 32.3 (3C), 31.4 (3C), 30.8, 30.0, 23.2, 13.9; **HRMS** (ESI+): exact mass calculated for [M+Na]⁺ (C₂₄H₃₇NO₄Na⁺) requires *m*/*z* 426.2615, found *m*/*z* 426.2606; **IR** (thin film): v 2957, 1774, 1708, 1384, 1217 cm⁻¹.

### 3-(2-(2,4-Dimethoxyphenyl)hexanoyl)oxazolidin-2-one

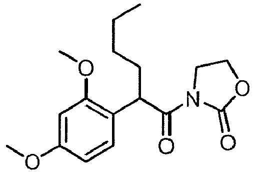

Performed according to general procedure on a 0.21 mmol scale delivering the product in 96% yield as white crystals.
**¹H-NMR** (400 MHz, CDCl₃): *δ* 7.18-7.07 (m, 1H), 6.50-6.41 (m, 2H), 5.22 (t, *J* = 7.3 Hz, 1H), 4.35 (td, *J* = 9.0, 6.4 Hz, 1H), 4.28 (td, *J* = 9.0, 7.4 Hz, 1H), 4.08-3.90 (m, 2H), 3.80 (s, 3H), 3.78 (s, 3H), 2.06-1.91 (m, 1H), 1.77-1.66 (m, 1H), 1.40-1.21 (m, 4H), 0.86 (t, *J* = 7.0 Hz, 3H); **¹³C-NMR** (101 MHz, CDCl₃): *δ* 175.1, 159.7, 158.2, 152.8, 128.5, 120.1, 104.2, 98.7, 61.6, 55.6, 55.2, 42.9, 41.7, 32.4, 29.6, 22.6, 13.9; **HRMS** (ESI+): exact mass calculated for [M+Na]⁺ (C₁₇H₂₃NO₅Na⁺) requires *m*/*z* 344.1468, found *m*/*z* 344.1467; **IR** (thin film): v 2934, 1777, 1694, 1506, 1384, 1208, 1038 cm⁻¹.

### 3-(2-(2, 6-Dimethoxyphenyl)acetyl)oxazolidin-2-one

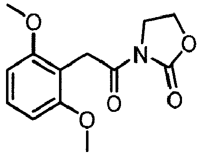

Performed on a 0.26 mmol scale delivering the product in 88% yield as white crystals.
**¹H-NMR** (400 MHz, CDCl₃): *δ* 7.22 (t, *J* = 8.3 Hz, 1H), 6.56 (d, *J* = 8.3 Hz, 2H), 4.43-4.37 (m, 2H), 4.28 (s, 2H), 4.08-3.98 (m, 2H), 3.78 (s, 6H); **¹³C-NMR** (101 MHz, CDCl₃): *δ* 171.3, 158.4 (2C), 153.9, 128.3, 110.8, 103.6 (2C), 62.1, 55.7 (2C), 42.6, 30.1; **HRMS** (ESI+): exact mass calculated for [M+Na]⁺ (C₁₃H₁₅NO₅Na⁺) requires *m*/*z* 288.0842, found *m*/*z* 288.0841; **IR** (thin film): *v* 2940, 1771, 1703, 1597, 1474, 1258, 1105, 731 cm⁻¹.

### Methyl 10-(2, 6-dimethoxyphenyl)-11-oxo-19-(2-oxooxazolidin-3-yl)undecanoate

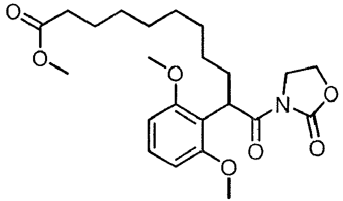

Performed on a 0.2 mmol scale delivering the product in 93% yield as colorless oil.
**¹H-NMR** (600 MHz, CDCl₃): *δ* 7.13 (t, *J* = 8.3 Hz, 1H), 6.51 (d, *J* = 8.3 Hz, 2H), 4.74 (t, *J* = 6.8 Hz, 1H), 4.29 (td, *J* = 9.2, 4.7 Hz, 1H), 4.17 (app q, *J* = 8.8 Hz, 1H), 4.06 (app dt, *J* = 18.3, 9.2 Hz, 1H), 3.84 (ddd, *J* = 10.6, 8.9, 4.7 Hz, 1H), 3.78 (s, 6H), 3.65 (s, 3H), 2.27 (t, *J* = 7.6 Hz, 2H), 2.13-2.03 (m, 1H), 1.62-1.55 (m, 3H), 1.39-1.19 (m, 10H); **¹³C-NMR** (151 MHz, CDCl₃): *δ* 175.0, 174.3, 157.9 (2C), 152.6, 127.9, 117.6, 104.4 (2C), 61.8, 55.9 (2C), 51.4, 43.2, 41.5, 34.1, 30.5, 29.6, 29.3, 29.2, 29.1, 27.2, 24.9; **HRMS** (ESI+): exact mass calculated for [M+Na]⁺ (C₂₃H₃₃NO₇Na⁺) requires m/z 458.2149, found m/z 458.2155; **IR** (thin film): *v* 2931, 1782, 1736, 1705, 1594, 1474, 1245, 1108 cm⁻¹.

### 2-(2,6-Dimethoxyphenyl)-1-(2-oxooxazolidin-3-yl)-11-phenylundecane-1,11-dione

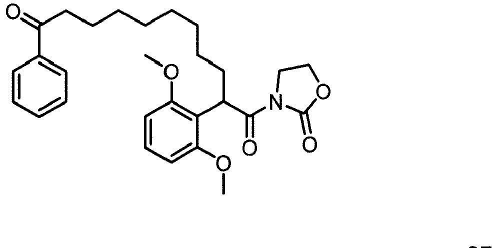

Performed on a 0.2 mmol scale delivering the product in 97% yield as colorless oil.
**¹H-NMR** (600 MHz, CDCl₃): *δ* 7.98-7.92 (m, 2H), 7.56-7.51 (m, 1H), 7.48-7.42 (m, 2H), 7.14 (t, *J* = 8.3 Hz, 1H), 6.52 (d, *J* = 8.3 Hz, 2H), 4.74 (t, *J* = 6.8 Hz, 1H), 4.29 (td, *J* = 9.2, 4.7 Hz, 1H), 4.18 (app q, *J* = 8.8 Hz, 1H), 4.04 (app dt, *J* = 18.4, 9.2 Hz, 1H), 3.85 (ddd, *J* = 10.6, 9.0, 4.7 Hz, 1H), 3.78 (s, 6H), 2.97-2.89 (m, 2H), 2.16-2.03 (m, 1H), 1.75-1.65 (m, 2H), 1.64-1.56 (m, 1H), 1.42-1.18 (m, 10H); **¹³C-NMR** (151 MHz, CDCl₃): *δ* 200.6, 175.0, 157.9 (2C), 152.7, 137.1, 132.8, 128.5 (2C), 128.0 (2C), 127.9, 117.5, 104.4 (2C), 61.8, 55.9 (2C), 43.2, 41.5, 38.6, 30.5, 29.6, 29.4, 29.3 (2C), 27.2, 24.4; **HRMS** (ESI+): exact mass calculated for [M+Na]⁺ (C₂₈H₃₅NO₆Na⁺) requires *m*/*z* 504.2357, found *m*/*z* 504.2358; **IR** (thin film): *v* 2931, 1782, 1704, 1687, 1594, 1474, 1240, 1108 cm⁻¹.

### 10-(2,6-Dimethoxyphenyl)-N-methoxy-N-methyl-11-oxo-11-(2-oxooxazolidin-3-yl)undecanamide

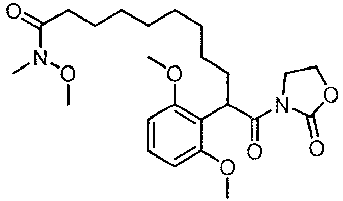

Performed on a 0.2 mmol delivering the product in 76% yield as colorless oil.
**¹H-NMR** (600 MHz, CDCl₃): *δ* 7.14 (t, *J* = 8.3 Hz, 1H), 6.52 (d, *J* = 8.3 Hz, 2H), 4.74 (t, *J* = 6.8 Hz, 1H), 4.30 (td, *J* = 9.0, 4.7 Hz, 1H), 4.18 (app q, *J* = 8.8 Hz, 1H), 4.06 (app dd, *J* = 19.6, 9.3 Hz, 1H), 3.85 (ddd, *J* = 10.6, 9.0, 4.7 Hz, 1H), 3.78 (s, 6H), 3.67 (s, 3H), 3.16 (s, 3H), 2.38 (t, *J* = 7.3 Hz, 2H), 2.13-2.04 (m, 1H), 1.63-1.56 (m, 3H), 1.42-1.19 (m, 10H); ¹³C-**NMR (151** MHz, CDCl₃): *δ* 194.9, 175.1, , 157.9 (2C), 152.6, 127.9, 117.6, 104.4 (2C), 61.8, 61.2, 55.9 (2C), 43.3, 41.5, 32.1, 31.9, 30.6, 29.7, 29.42, 29.37 (2C), 27.3, 24.6; **HRMS** (ESI+): exact mass calculated for [M+Na]⁺ (C₂₄H₃₆N₂O₇Na⁺) requires m/z 487.2415, found *m*/*z* 487.2413; **IR** (thin film): *v* 2930, 1781, 1704, 1660, 1474, 1384, 1107 cm⁻¹.

### Benzyl (S)-2-((tert-butoxycarbonyl)amino)-5-(2,6-dimethoxyphenyl)-6-oxo-6-(2-oxooxazolidin-3-yl)hexanoate

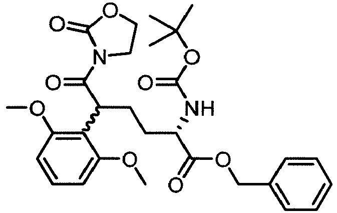

Performed on a 0.1 mmol scale delivering the product in 89% yield as a 1.1:1 mixture of diastereomers as colorless oil.
**¹H-NMR** (500 MHz, CDCl₃): *δ* 7.40-7.22 (m, 5H+5H), 7.19-7.11 (m, 1H+1H), 6.61-6.42 (m, 2H+2H), 5.25-4.79 (m, 3H+3H), 4.79-4.69 (m, 1H+1H), 4.47-4.23 (m, 2H+2H), 4.22-4.13 (m, 1H+1H), 4.10-3.97 (m, 1H+1H), 3.92-3.64 (m, 7H+7H), 2.20-2.08 (m, 1H+1H), 1.93-1.80 (m, 1H+1H), 1.79-1.64 (m, 2H+2H), 1.46-1.32 (m, 9H+9H); **¹³C-NMR** (126 MHz, CDCl₃): *δ* 174.5, 174.4, 172.84, 172.80, 157.8 (2C), 157.7 (2C), 155.5 (2C), 152.5 (2C), 135.6, 135.5, 128.5 (2C), 128.4 (2C), 128.3 (2C + 2C), 128.13, 128.11, 128.08, 128.0, 116.4, 116.2, 104.4 (2C), 104.3 (2C), 79.64, 79.60, 66.8 (2C), 61.9 (2C), 55.9 (2C), 55.8 (2C), 53.9, 53.5, 43.2 (2C), 41.2, 40.7, 29.7, 29.4, 28.3 (3C + 3C), 26.4, 26.0; **HRMS** (ESI+): exact mass calculated for [M+Na]⁺ (C₂₉H₃₆N₂O₉Na⁺) requires *m*/*z* 579.2313, found *m*/*z* 579.2318; IR (thin film): *v* 3367, 2972, 1782, 1741, 1705, 1594, 1475, 1386, 1365, 1167, 1107, 732 cm⁻¹.

### 2-(2,6-Dimethoxyphenyl)-N-phenyl-2-(pyridin-2-yl)-N-tosylacetamide

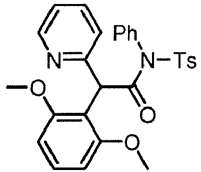

Performed on a 0.05 mmol scale using 1.2 equiv of Tf₂NH delivering the product in 45% yield.
**¹H-NMR** (400 MHz, CDCl₃): *δ* 8.39-8.34 (m, 2H), 7.87 (d, *J* = 8.3 Hz, 2H), 7.75 (dt, *J* = 8.0, 1.9 Hz, 1H), 7.32-7.26 (m, 2H), 7.37-6.44 (m br, 5H), 7.14 (ddd, *J* = 8.0, 4.8, 0.6 Hz, 1H), 7.10 (t, *J* = 8.3 Hz, 1H), 6.31 (d, *J* = 8.4 Hz, 2H), 5.08 (s, 1H), 3.57 (s, 6H), 2.43 (s, 3H); **¹³C-NMR** (101 MHz, CDCl₃): *δ* 172.6, 157.6 (2C), 150.6, 148.1, 144.4, 137.7, 136.4, 135.7, 133.7, 129.4 (2C), 129.3, 129.2 (2C), 128.7, 128.3 (2C), 123.2, 114.3, 103.3 (2C), 55.3 (2C), 45.5, 21.6, ortho-carbon atoms of *N*-Ph not observed; **HRMS** (ESI+): exact mass calculated for [M+H]⁺ (C₂₈H₂₇N₂O₅S⁺) requires *m*/*z* 503.1635, found *m*/*z* 503.1635; **IR** (thin film): *v* 1712, 1595, 1476, 1360, 1245, 1172, 1154, 1107, 696 cm⁻¹.

### 2-Chloro-2-(2,6-dimethoxyphenyl)-N-phenyl-N-tosylacetamide

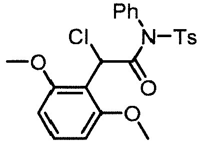

Performed on a 0.1 mmol scale using 30 mol% of Tf₂NH delivering the product in 63% as white solid.
**¹H-NMR** (400 MHz, CDCl₃): *δ* 7.97-7.87 (m, 2H), 7.33 (d, *J* = 8.0 Hz, 2H), 7.30-7.25 (m, 1H), 7.17 (t, *J* = 8.4 Hz, 1H), 7.12 (s br, 2H), 6.70 (s br, 2H), 6.30 (d, *J* = 8.4 Hz, 2H), 5.78 (s, 1H), 3.54 (s, 6H), 2.45 (s, 3H); **¹³C-NMR** (101 MHz, CDCl₃): *δ* 167.6, 157.8 (2C), 144.8, 135.9, 134.9, 131.2, 129.6 (2C), 129.3 (2C), 129.0, 128.6 (2C), 112.7, 103.3 (2C), 55.5 (2C), 54.2, 21.7, ortho-carbon atoms of *N*-Ph not observed; **HRMS** (ESI+): exact mass calculated for [M+Na]⁺ (C₂₃H₂₂NO₅SClNa⁺) requires *m*/*z* 482.0799, found *m*/*z* 482.0798; **IR** (thin film): *v* 1730, 1597, 1479, 1363, 1259, 1188, 1108, 695 cm⁻¹.

### S-methyl 2-(2,6-dimethoxyphenyl)hexanethioate

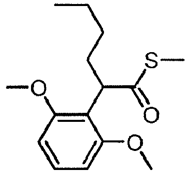

The solution of thioalkyne (25.6 mg, 0.2 mmol, 1.0 equiv) and sulfoxide (48.1 mg, 0.24 mmol, 1.2 equiv) in CH₂Cl₂ (2 mL, 10 mL mmol ⁻¹) was cooled to 0 °C in an ice bath, octanethiol (35 µL, 0.2 mmol, 1 equiv) and a solution of Tf₂NH in CH₂Cl₂ (1 mL of a 0.1 M solution, 0.1 mmol, 0.5 equiv) were added and reaction was stirred at 0 °C for 3 h. Solid NaHCO₃ (20 mg) was added and the mixture was filtered, evaporated *in vacuo* and purified by column chromatography (heptane/ethyl acetate 100:0 to 90:10) to give the desired product in 77% yield (43.2 mg) as a colorless oil.
**¹H-NMR** (400 MHz, CDCl₃): *δ* 7.24 (t, *J* = 8.3 Hz, 1H), 6.57 (d, *J* = 8.3 Hz, 2H), 4.28 (dd, *J* = 9.3, 5.0 Hz, 1H), 3.79 (s, 6H), 2.31-2.14 (m, 4H), 1.83 (dtd, *J* = 14.6, 9.6, 5.2 Hz, 1H), 1.39-1.13 (m, 3H), 1.13-0.99 (m, 1H), 0.83 (t, *J* = 7.2 Hz, 3H); **¹³C-NMR** (101 MHz, CDCl₃): *δ* 202.1, 158.8 (2C), 128.8, 116.4, 104.3 (2C), 55.8 (2C), 49.2, 29.6, 28.7, 22.5, 13.9, 11.6; **HRMS** (ESI+): exact mass calculated for [M+Na]⁺ (C₁₅H₂₂O₃SNa⁺) requires m/z 305.1182, found *m*/*z* 305.1187; **IR** (thin film): *v* 2956, 2930, 1692, 1594, 1474, 1251, 1101, 788 cm⁻¹.

### 2-(2,6-Dimethoxyphenyl)-1-(4-methoxyphenyl)ethan-1-one

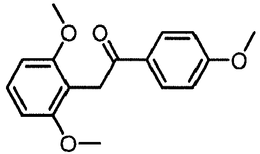

To a solution of alkyne (26.4 mg, 0.2 mmol, 1.0 equiv) and sulfoxide (44.1 mg, 0.22 mmol, 1.1 equiv) in nitromethane (0.5 mL, 2.5 mL mmol⁻¹) were added octanethiol (38 µL, 0.22 mmol, 1.1 equiv) and triflic acid (8 µL, 0.09 mmol, 45 mol%). The solution was stirred at room temperature for 14 h. Solid NaHCO₃ (30 mg) was added and the reaction was further stirred for 5 min before filtration and evaporation of the solvent. Purification by column chromatography (heptane/ethyl acetate 9:1 to 1:1) afforded the desired compound in 67% yield (38.3 mg) as yellow crystals.
**¹H-NMR** (400 MHz, CDCl₃): *δ* 8.07-7.97 (m, 2H), 7.21 (t, *J* = 8.3 Hz, 1H), 6.97-6.90 (m, 2H), 6.57 (d, *J* = 8.3 Hz, 2H), 4.29 (s, 2H), 3.87 (s, 3H), 3.76 (s, 6H); **¹³C-NMR** (101 MHz, CDCl₃): *δ* 196.5, 163.1, 158.4 (2C), 130.6, 130.4 (2C), 128.1, 113.5 (2C), 112.6, 103.8 (2C), 55.8 (2C), 55.4, 33.6; **HRMS** (ESI+): exact mass calculated for [M+Na]⁺ (C₁₇H₁₈O₄Na⁺) requires *m*/*z* 309.1097, found *m*/*z* 309.1098; **IR** (thin film): *v* 2918, 1673, 1596, 1573, 1474, 1260, 1174, 1099, 1027, 787 cm⁻¹.

### Ethyl 2-(2,6-dimethoxyphenyl)acetate

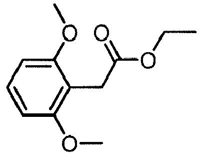

Performed on a 0.5 mmol scale using a solution ethoxyethyne (0.14 mL, 50% in toluene, 1.5 equiv) delivering the desired product in 67% yield as colorless oil.
**¹H-NMR** (600 MHz, CDCl₃): *δ* 7.20 (t, *J* = 8.3 Hz, 1H), 6.56 (d, *J* = 8.3 Hz, 2H), 4.15 (q, *J* = 7.1 Hz, 2H), 3.80 (s, 6H), 3.69 (s, 2H), 1.25 (t, *J* = 7.1 Hz, 3H); **¹³C-NMR** (151 MHz, CDCl₃): *δ* 172.2, 158.3 (2C), 128.1, 111.5, 103.6 (2C), 60.3, 55.7 (2C), 28.8, 14.2; **HRMS** (ESI+): exact mass calculated for [M+Na]⁺ (C₁₂H₁₆O₄Na⁺) requires *m*/*z* 247.0941, found *m*/*z* 247.0939; **IR** (thin film): *v* 2923, 1734, 1598, 1474, 1258, 1106, 778 cm⁻¹.

## Claims

1. A method for the preparation of an alpha-substituted carbonyl compound or an alpha-substituted nitrile compound carrying an aryl or heteroaryl group as an alpha-substituent, said method comprising reacting
(i) a sulfoxide compound S1 comprising a substituted aryl or heteroaryl group, wherein a sulfinyl moiety is attached to a carbon ring atom of an aromatic ring,
with a reactant selected from the following reactants A1, A2 and A3:
(ii-1) an alkyne compound A1, which is selected from an ynamide A1a, an ynolether A1b, a thioalkyne A1c and an arylalkyne A1d, in the presence of an acid selected from a Bronsted- and a Lewis acid;
(ii-2) a carbonyl compound A2, which is selected from a tertiary carboxylic acid amide A2a which carries at least one hydrogen atom attached to a carbon atom in alpha-position to its carbonyl group and an aryl ketone A2b which carries at least one hydrogen atom attached to a carbon atom in alpha-position to its carbonyl group, in the presence of an electrophilic activator; and
(ii-3) a nucleophile A3, which is selected from a silyl enolether A3a, an α-silyl nitrile A3b, an α-stannyl nitrile A3c, and a β-ketoester derivative A3d, in the presence of an electrophilic activator;
to yield an alpha-substituted carbonyl compound or nitrile compound wherein a carbon atom in alpha-position to the carbonyl group or the nitrile group, respectively, forms a bond with the carbon ring atom of the aromatic ring to which the sulfinyl moiety had been attached in the sulfoxide compound S1.

2. The method of claim 1, wherein the aryl group or heteroaryl group comprised by S1 contains a monocyclic 5- or 6-membered aromatic ring, and a sulfinyl moiety attached to a carbon ring atom of the aromatic ring, or a 9- or 10-membered bicyclic aromatic ring system, and a sulfinyl moiety attached to a carbon ring atom of an aromatic ring forming part of the ring system.

3. The method of claim 1 or 2, wherein the sulfinyl group is provided by a substituent -S(O)-R^{1S} which is attached to a carbon ring atom of an aromatic ring, wherein R^{1S} is a hydrocarbyl group, preferably a C1-C6 alkyl group.

4. The method of any of claims 1 to 3, wherein the sulfoxide compound is a sulfoxide compound wherein the sulfinyl moiety is attached to a carbon ring atom of a 6-membered carbocyclic aromatic ring which may be part of a fused aromatic ring system, and the 6-membered carbocyclic aromatic ring carries at least two further substituents in addition to the sulfinyl moiety, namely:
(i) an electron donating substituent selected from -OH, -OR^{1D}, -NH₂, -NHR^{2D} and -NR^{2D}R^{3D} in *ortho* or *para*-position relative to the sulfinyl moiety, wherein R^{1D}, R^{2D} and R^{3D} are independently a hydrocarbyl group;
and
(ii) a substituent in *ortho*-position relative to the sulfinyl moiety, which may be linked with a further substituent carried by the 6-membered aromatic ring to form a ring which is fused to the 6-membered aromatic ring.

5. The method of claim 4, wherein the 6-membered carbocyclic aromatic ring of the preferred sulfoxide compound carries at least two further substituents which are both an electron donating substituent selected from -OH, -OR^{1D}, -NH₂, -NHR^{2D} and -NR^{2D}R^{3D}, wherein R^{1D}, R^{2D} and R^{3D} are independently a hydrocarbyl group, one of them being in *ortho-*position and the other one being in *ortho* or para-position relative to the sulfinyl moiety.

6. The method of any of claims 1 to 3, wherein the sulfoxide compound is a sulfoxide compound wherein the sulfinyl moiety is attached to a carbon ring atom of a 5-membered heterocyclic aromatic ring which may be part of a fused aromatic ring system, and wherein the 5-membered carboxylic aromatic ring carries at least one further substituent in *ortho-*position relative to the sulfinyl moiety, which may be linked with a further substituent on the 5-membered aromatic ring to form a ring which is fused to the 5-membered aromatic ring.

7. The method of claim 6, wherein the 5-membered heterocyclic aromatic ring either
(i) carries at least two substituents, which are attached to carbon ring atoms in *ortho-*position to the sulfinyl moiety and which may be linked with a further substituent on the 5-membered aromatic ring to form a ring which is fused to the 5-membered aromatic ring; or
(ii) carries one substituent in *ortho*-position to the sulfinyl moiety, which may be linked with a further substituent on the 5-membered aromatic ring to form a ring which is fused to the 5-membered aromatic ring, and the 5-membered aromatic ring additionally has a heteroatom as a ring member adjacent to the carbon atom to which the sulfinyl moiety is attached.

8. The method of any of claims 1 to 7, wherein the sulfoxide compound S1 is reacted with an alkyne compound A1 selected from:
a) an ynamide represented by formula (2) or (3) 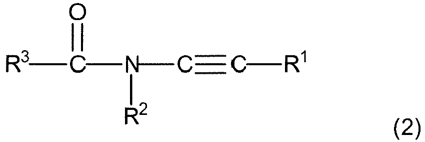 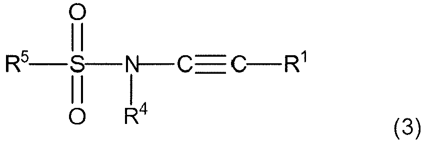 wherein
R¹ is selected from hydrogen, an organyl group, and halogen; R² and R⁴ are an organyl group; R³ is selected from an organyl group and an oxyorganyl group; R⁵ is an organyl group; and R² and R³ may be linked to form a ring together with the atoms to which they are attached;
b) an ynolether or a thioalkyne represented by formula (4) or (5)
R⁶-O-C≡C-R¹ (4)
R⁷-S-C≡C-R¹ (5)
wherein
R¹ is selected from hydrogen, an organyl group, an oxyorganyl group, and halogen; and R⁶ and R⁷ are an organyl group; and
c) an arylalkyne represented by formula (6)
Ar¹-C≡C-R¹ (6)
wherein
R¹ is selected from hydrogen, an organyl group and halogen; and Ar¹ is an optionally substituted aryl group.

9. The method of any of claims 1 to 8, wherein the sulfoxide compound S1 is reacted with the alkyne compound A1, and the reaction is carried out in the presence of a Brønsted acid having a pKa of less than -2.5.

10. The method of any of claims 1 to 7, wherein the sulfoxide compound S1 is reacted with a carbonyl compound A2 selected from:
a) a tertiary carboxylic acid amide represented by formula (7) 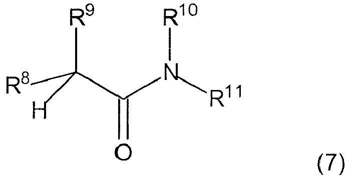 wherein
R⁸ and R⁹ are independently selected from hydrogen and an organyl group; and R¹⁰ and R¹¹ are independently selected from hydrogen and an organyl group; and
b) an aryl ketone represented by formula (8) 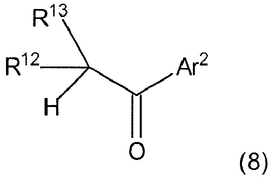 wherein
Ar² is an optionally substituted aryl group, e.g. an optionally substituted phenyl or naphthyl group; and R¹² and R¹³ are independently selected from hydrogen and an organyl group.

11. The method of any of claims 1 to 7 and 10, wherein the sulfoxide compound S1 is reacted with a carbonyl compound A2 in the presence of trifluoromethanesulfonic acid anhydride as an electrophilic activator.

12. The method of any of claims 1 to 7, wherein the sulfoxide compound S1 is reacted with a nucleophile A3 selected from:
a) a silyl enolether represented by formula (9) 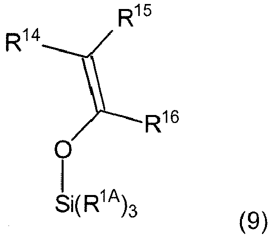 wherein
R^{1A}, independently for each occurrence, is a hydrocarbyl group, and R¹⁴ to R¹⁶ are independently selected from hydrogen and an organyl group;
b) an α-silyl nitrile or an α-stannyl nitrile represented by formula (10) 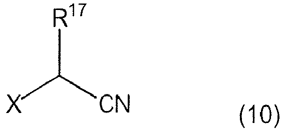 wherein
X is selected from -Si(R^{2A})₃ and -Sn(R^{3A})₃, R^{2A} and R^{3A} are, independently for each occurrence, a hydrocarbyl group, and R¹⁷ is selected from hydrogen, and an organyl group; and
c) a β-ketoester derivative represented by formula (11) 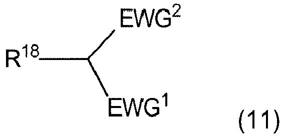 wherein
EWG¹ and EWG² are the same or different, and are selected from -C(O)R^{4A}, -C(O)OR^{5A}, -C(O)NR^{6A}R^{7A}, and CN, with R^{4A}, R^{5A}, R^{6A} and R^{7A} being independently selected from hydrogen and a hydrocarbyl group; and R¹⁸ is selected from hydrogen and an organyl group.

13. The method of any of claims 1 to 7 and 12, wherein the sulfoxide compound S1 is reacted with the nucleophile A3 in the presence of an electrophilic activator selected from trifluoromethanesulfonic acid anhydride, trifluoroacetic acid anhydride, toluenesulfonic anhydride, methanesulfonic anhydride, and TfOC(O)CF₃.

14. The method of any of claims 1 to 13, wherein the sulfoxide compound S1 is reacted with the alkyne compound A1, the carbonyl compound A2 or the nucleophile A3 in the further presence of a soft nucleophile as an additive.

15. The method of claim 14, wherein the soft nucleophile is selected from octanethiol, phenylthiol, 2-methyl-2-butene, and 2,3-dimethylbut-2-ene.
